(19) Europäisches Patentamt
European Patent Office
Office européen des brevets

(11) Veröffentlichungsnummer: **0 029 990**
**B1**

# (12) EUROPÄISCHE PATENTSCHRIFT

(45) Veröffentlichungstag der Patentschrift: **08.09.82**

(51) Int. Cl.³: **C 07 C 143/50,** C 07 C 139/00, A 61 K 31/185

(21) Anmeldenummer: **80107328.9**

(22) Anmeldetag: **25.11.80**

(54) **Diphenylmethanderivat, Verfahren zu seiner Herstellung und es enthaltende Arzneimittel.**

(30) Priorität: **04.12.79 CH 10763/79**

(43) Veröffentlichungstag der Anmeldung: **10.06.81 Patentblatt 81/23**

(45) Bekanntmachung des Hinweises auf die Patenterteilung: **08.09.82 Patentblatt 82/36**

(84) Benannte Vertragsstaaten: **AT BE CH DE FR GB IT LI LU NL SE**

(56) Entgegenhaltungen: **DE-B-1 518 102**

(73) Patentinhaber: **Byk Gulden Lomberg Chemische Fabrik GmbH, Byk-Gulden-Strasse 2, D-7750 Konstanz (DE)**

(72) Erfinder: **Klemm, Kurt, Prof. Dr., Im Weinberg 2, D-7753 Allensbach (DE)**
Erfinder: **Haerlin, Rüdiger, Dr., Hohenhewenstrasse 7, D-7760 Radolfzell (DE)**
Erfinder: **Zick, Franz, Haidelmoosweg 36, D-7750 Konstanz (DE)**
Erfinder: **Baron, Lothar, Freiherr von Hundbisstrasse 2, D-7752 Reichenau (DE)**
Erfinder: **Prüsse, Wolfgang, Dr., Konstanzer Strasse 13, D-7753 Allensbach (DE)**
Erfinder: **Krüger, Uwe, Dr., Neuhauser Strasse 11, D-7750 Konstanz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTCRUM AG

EP 0 029 990 B1

# Diphenylmethanderivat, Verfahren zu seiner Herstellung und es enthaltende Arzneimittel

Die Erfindung betrifft die Verbindung 2,2′-Dihydroxy-5,5′-methylendi-(p-toluolsulfonsäure) und ihre Salze, Verfahren zur ihrer Herstellung und sie enthaltende Arzneimittel.

Gerbend wirkende Kondensationsprodukte aus Kresolsulfonsäuren mit Formaldehyd sind seit vielen Jahre bekannt und wurden erstmals in der DE-PS-262 558 beschrieben. In der US-PS-2 326 578 wird für ein durch Umsetzung von Metakresolsulfonsäuren mit Formaldehyd erhältliches gerbendes Kondensationsprodukt erwähnt, dass es sich auch zur Heilbehandlung von krankem Gewebe eignen soll. Dies führte in der Folgezeit zur Entwicklung von Arzneimitteln auf der Basis von Kondensationsprodukten aus m-Kresolsulfonsäuren und Formaldehyd, insbesondere auf dem Gebiet der Gynäkologie. Ein entsprechendes Arzneimittel hat sich aufgrund seiner hervorragenden heilenden Eigenschaften zu einem weitverbreiteten Produkt (Albothyl®, Lotagen®, Negatol®) entwickelt. Wie durch zahlreiche Untersuchungen nachgewiesen werden konnte, wirkt es adstringierend, bakterizid, trichomonazid, fungizid, hämostyptisch und besitzt eine selektive Wirkung auf abgestorbenes sowie krankhaft verändertes Gewebe, das koaguliert und abgestossen wird, wobei gesundes Gewebe nicht angegriffen wird. Es wird deshalb nicht nur in vielfältiger Weise in der Frauenheilkunde, sondern auch in der Urologie, der Chirurgie, der Hals-, Nasen-, Ohrenheilkunde, der Gastroenterologie, der Proktologie und bei Hauterkrankungen mit grossem Erfolg eingesetzt.

Nachteilig an diesem Produkt ist, dass dessen wirksames Prinzip aus einem Gemisch von zahlreichen Stoffen besteht. Bei dem sehr komplexen Reaktionsablauf der Kondensation von m-Kresolsulfonsäuren mit Formaldehyd entstehen nämlich eine Vielzahl von verschiedenen Verbindungen. Es ist bisher trotz Einsatz modernster Untersuchungsmethoden nicht gelungen, die genaue Zusammensetzung zu ermitteln. Da es für ein Arzneimittel unerlässlich ist, dass dieses immer die gleiche Zusammensetzung aufweist, bedarf es bei der Herstellung des Kondensationsproduktes aus m-Kresolsulfonsäure und Formaldehyd sehr grosser Sorgfalt bei der Reaktionsführung. Eine Abweichung von der empirisch optimierten Gemischzusammensetzung wäre auch deshalb sehr bedenklich, weil bei der genannten Kondensationsreaktion leicht Produkte entstehen können, die sich später langsam in unkontrollierbarer Weise chemisch zersetzen bzw. umwandeln, was insbesondere die Lagerstabilität beeinträchtigen würde. Ein weiterer Nachteil des Handelsproduktes liegt in seiner intensiven dunkelbraunen Färbung, die auf manche Patienten erfahrungsgemäss abstossend wirkt. Ausserdem lässt sich bei der Anwendung trotz sorgfältiger Vorkehrungen eine Verschmutzung und Beschädigung von Leib- und Bettwäsche nicht immer verhindern.

Um diese Nachteile des aus therapeutischer Sicht sehr wertvollen Arzneimittels zu beseitigen, wurden grosse Anstrengungen unternommen: In der DE-PS-1 031 799 wird die Herstellung eines kristallinen wasserlöslichen Kondensationsproduktes aus sulfoniertem m-Kresol mit Formaldehyd offenbart. In den DE-OS-2 245 411 und 2 444 785 werden oligomere Kondensationsprodukte aus p-Kresolsulfonsäure und Formaldehyd der allgemeinen Formel

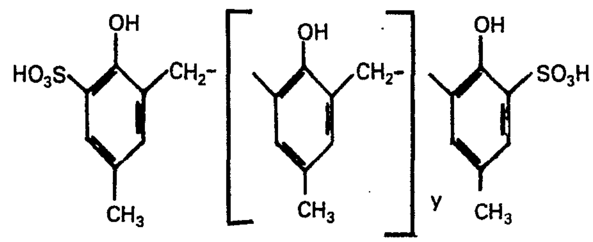

beschrieben, worin y die Werte 0, 1, 2 und 3 annehmen kann. Diesen Anstrengungen war allerdings bisher kein Erfolg beschieden, da die vorgeschlagenen Produkte entweder wirtschaftlich nicht vertretbaren Aufwand bei der Herstellung erforderten und/oder nicht die gewünschte Stabilität und/oder vor allem in ihrer Wirkungsqualität und -quantität nicht an das Handelsprodukt heranreichten.

Es wurde nun überraschend gefunden, dass die neue Verbindung 2,2′-Dihydroxy-5,5′-methylendi-(p-toluolsulfonsäure)

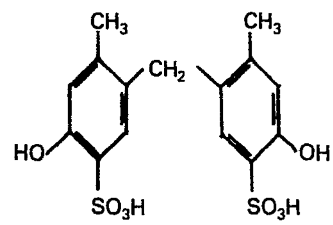

und ihre Salze mit organischen und anorganischen Basen unproblematisch herzustellen sind und dem handelsüblichen Kondensationsprodukt aus Metakresolsäuren mit Formaldehyd in seiner therapeutischen Wirkung bei geringerer Toxizität ebenbürtig sind.

Gegenstand der Erfindung ist die Verbindung 2,2′-Dihydroxy-5,5′-methylendi-(p-toluolsulfonsäure)

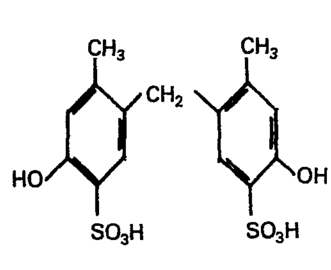

und ihre Salze. Die neue Verbindung kann auch als 5,5′-Methylendi-(2-hydroxy-p-toluolsulfonsäure) bezeichnet werden.

Als Salze kommen Salze mit anorganischen und organischen Basen in Betracht. Pharmakologisch nicht verträgliche Salze werden nach an sich bekannten Methoden in pharmakologisch verträgliche Salze übergeführt, die unter den erfindungsgemässen Salzen bevorzugt sind. Als Kationen für die Salzbildung werden vor allem die Kationen der Alkalimetalle oder der leichten Erdalkalimetalle verwendet; es kommen jedoch auch die entsprechenden Kationen organischer Stickstoffbasen, wie Amine, Aminoalkanole, etc. zur Anwendung.

Beispielsweise seien die Salze von Lithium, Natrium, Kalium, Magnesium, Calcium, Morpholin, Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Harnstoff, Tetramethylharnstoff, 1,3-Dimethylimidazolidinon-2, Glucamin, N-Methylglucamin, Glucosamin, N-Methylglucosamin, Lysin, Ornithin, Arginin, Chinolin genannt.

Bevorzugte Salze sind solche, die in Wasser eine gute Löslichkeit aufweisen. Als gut löslich werden in diesem Zusammenhang Salze verstanden, die bei 23 °C in Wasser zu mehr als 3 g/100 ml, vorzugsweise zu mehr als 10 g/100 ml löslich sind. Beispiele für bevorzugte gut lösliche Salze sind die Alkalisalze, insbesondere das Natriumsalz, das Ammoniumsalz und Salze mit Harnstoff.

Ein weiterer Gegenstand der Erfindung ist ein Verfahren zur Herstellung der Verbindung 2,2'-Dihydroxy-5,5'-methylendi-(p-toluolsulfonsäure) und ihrer Salze, das dadurch gekennzeichnet ist, dass man

a) m-Kresol-6-sulfonsäure und/oder deren Salze in saurem Medium mit Formaldehyd und/oder Formaldehyd abspaltenden Mitteln umsetzt und gewünschtenfalls ein erhaltenes Salz in die freie Säure oder ein anderes Salz überführt,

b) m-Kresol-6-sulfonsäure und/oder deren Salze in basischem Medium mit Formaldehyd und/oder Formaldehyd abspaltenden Mitteln in 4-Hydroxymethyl-m-kresol-6-sulfonsäure überführt und diese in saurem Medium mit m-Kresol-6-sulfonsäure und/oder deren Salzen kondensiert und gewünschtenfalls ein erhaltenes Salz in die freie Säure oder ein anderes Salz überführt, oder

c) in 6-Stellung mit einem abspaltbaren Substituenten substituiertes m-Kresol in saurem Medium mit Formaldehyd und/oder Formaldehyd abspaltenden Mitteln zu 5,5'-disubstituiertem 4,4'-Dihydroxy-2,2'-dimethyldiphenylmethan umsetzt, die Substituenten in 5- und 5'-Stellung abspaltet und in saurem Medium mit Schwefelsäure und/oder einem Sulfonsäuregruppen abspaltenden Mittel sulfoniert und gewünschtenfalls ein erhaltenes Salz in die freie Säure oder ein anderes Salz überführt.

Die Umsetzung mit Formaldehyd und Formaldehyd abspaltenden Mitteln führt man vorzugsweise in wässriger Lösung durch. Formaldehyd wird in Form von wässrigen Lösungen mit einem Gehalt von 10 bis 50 Gewichtsprozent, vorzugsweise 35 bis 50 Gewichtsprozent Formaldehyd eingesetzt. Als Formaldehyd abspaltende Mittel kommen neben Hexamethylentetramin insbesondere die Oligomeren (z.B. Trioxan, Tetroxan) und die Polymeren (z.B. Paraformaldehyd) des Formaldehyds in Frage, wobei Paraformaldehyd bevorzugt ist. Die Kondensationsreaktion mit Formaldehyd wird bei Temperaturen von 10 bis 90 °C, vorzugsweise bei Temperaturen von 20 bis 60 °C durchgeführt. Die Reaktionsdauer für die Umsetzung mit Formaldehyd ist abhängig von der Temperatur und davon abhängig, als welches Salz der Kondensationspartner vorliegt. Die Reaktionsdauer wird zweckmässig so gewählt, dass die Bildung des gewünschten Diphenylmethanderivates möglichst vollständig, eine Bildung von höheren oligomeren Kondensationsprodukten jedoch in möglichst geringem Mass eintritt. Je nach gewählter Temperatur und Art des eingesetzten Salzes des Kresolderivates ergeben sich für den Fachmann durch einfache Vorversuche optimale Reaktionszeiten zwischen wenigen Stunden und mehreren Tagen.

Als Umsetzung in saurem Medium wird eine Umsetzung in Gegenwart von starken Säuren, wie Halogenwasserstoffsäuren, vorzugsweise Salzsäure, Schwefelsäure, Sulfonsäure, Phosphorsäure, sauren Kationenaustauschern oder dergleichen verstanden. Bei der Umsetzung von m-Kresol-6-sulfonsäure mit Formaldehyd ist es nicht notwendig, eine starke Säure einzusetzen, da m-Kresol-6-sulfonsäure selbst eine starke Säure ist.

Als Umsetzung in basischem Medium wird eine Umsetzung in Gegenwart von starken Basen, vorzugsweise Alkalihydroxiden, aber auch Erdalkalihydroxiden, vorzugsweise Calciumhydroxid, verstanden.

Als abspaltbare Substituenten für in 6-Stellung mit einem abspaltbaren Substituenten substituiertes m-Kresol in Verfahrensvariante c) kommen Chlor, Brom und Iod, vorzugsweise Chlor in Frage. Bevorzugt sind solche Substituenten, die nach der Umsetzung des in 6-Stellung substituierten m-Kresol mit Formaldehyd zu 5,5'-disubstituiertem 4,4'-Dihydroxy-2,2'-dimethyl-diphenylmethan leicht durch Reduktion abgespalten werden können. Verwendet man als abspaltbare Substituenten Chlor, Brom oder Iod, so wird die reduktive Abspaltung vorzugsweise in an sich bekannter Weise durch katalytische Hydrierung bewerkstelligt, wobei die für diese Zwecke üblichen Hydrierungskatalysatoren wie Platin-, Palladium- und Nickelkatalysatoren, vorzugsweise Platinkatalysatoren verwendet werden. Als Lösungsmittel für die reduktive Abspaltung der abspaltbaren Substituenten kommen die für katalytische Hydrierungen üblichen in Frage, beispielsweise Wasser, Methanol, Ethanol, Essigester, Eisessig oder Gemische dieser Lösungsmittel. Es ist dem Fachmann bekannt, die Bedingungen für die Einwirkung von Wasserstoff so zu wählen, dass zwar die gewünschte Abspaltung des abspaltbaren Substituenten, nicht aber eine Hydrierung der aromatischen Kerne erfolgt.

Die Sulfonierung von 4,4'-Dihydroxy-2,2'-dimethyl-diphenylmethan zu 2,2'-Dihydroxy-5,5'-methylendi-(p-toluolsulfonsäure) erfolgt in an sich bekannter Weise mit 50 bis 100%iger, vorzugsweise 70 bis 100%iger Schwefelsäure. Besonders schonend kann die Sulfonierung mittels Schwefelsäure oder Schwefeltrioxid in Lösungsmitteln wie Chloroform oder flüssigem Schwefeldioxid durchgeführt werden. Als Sulfonsäuregruppen abspaltende Mittel kommen weiterhin beispielsweise Chlorsulfonsäure und Amidosulfonsäure in Frage.

Das bei der Umsetzung von m-Kresol-6-sulfonsäure mit Formaldehyd entstehende Reaktionsgemisch kann in üblicher Weise durch Zugabe von zur Ausfällung des gewünschten Reaktionsproduktes geeigneten Lösungsmitteln aufgearbeitet werden, indem das ausgefällte Reaktionsprodukt abfiltriert wird. Als sehr vorteilhaft hat es sich jedoch erwiesen, alternativ das gewünschte Reaktionsprodukt als schwerlösliches Salz auszufällen. Als Salze eignen sich die der schwerlöslichen Erdalkali- und Erdmetalle, wobei die der Erdalkalimetalle, insbesondere des Bariums, bevorzugt sind. Besonders bevorzugt ist die Verwendung organischer Amine, wie beispielsweise Mono-, Di- und Trialkylamine, p-Toluidin, α- und β-Naphthylamin, m-Nitranilin, β-Phenylethylamin, Di-o-tolylguanidin, Benzidin, Dianisidin, Dimethylanilin, m-Phenylendiamin, Pyridin, Morpholin und ähnlicher Amine. Unter organischen Aminen ist die Verwendung von Diaminen bevorzugt, von denen solche bevorzugt sind, in denen die Amingruppen durch eine, zwei oder drei $C_2$ bis $C_4$-Alkylketten, insbesondere Ethylketten, verbunden sind. Bevorzugte Diamine sind beispielsweise Piperazin, Diazabicyclo[2.2.2]oktan, 1,4-Di-$C_1$ bis $C_4$-alkyl-, insbesondere 1,4-Dimethylpiperazin, 1-Benzyl-4-methylpiperazin, Ethylendiamin, Di- oder Tetra-$C_1$ bis $C_4$-alkyl-, insbesondere Tetramethylethylendiamin.

Anstatt 2,2'-Dihydroxy-5,5'-methylendi-(p-toluolsulfonsäure) durch Zugabe der entsprechenden Basen in Form ihrer Salze auszufällen, besteht alternativ die Möglichkeit, die entsprechenden Salze der m-Kresol-6-sulfonsäure der Kondensation mit Formaldehyd zu unterwerfen, wobei die entsprechenden schwerlöslichen Salze der 2,2'-Dihydroxy-5,5'-methylendi-(p-toluolsulfonsäure) direkt entstehen.

Alternativ besteht die Möglichkeit, die Kondensation von m-Kresol-6-sulfonsäure bzw. eines löslichen Salzes der m-Kresol-6-sulfonsäure, wie z.B. des Ammonium- oder Natriumsalzes, mit Formaldehyd in Gegenwart von mit 2,2'-Dihydroxy-5,5'-methylendi-(p-toluolsulfonsäure) schwerlösliche Salze bildenden Basen, wobei die obengenannten bevorzugt sind, durchzuführen.

Im Rahmen des erfindungsgemässen Verfahrens zur Herstellung von 2,2'-Dihydroxy-5,5'-methylendi-(p-toluolsulfonsäure) ist insbesondere die Verwendung von solchen mit dieser Verbindung schwerlösliche Salze bildenden Basen vorteilhaft, die mit m-Kresol-6-sulfonsäure Salze bilden, die eine grössere Löslichkeit aufweisen. Bevorzugt werden Basen verwendet, deren Salze mit m-Kresol-6-sulfonsäure bei 23 °C eine Löslichkeit in Wasser aufweisen, die 5 bis 50 mal, vorzugsweise 5 bis 20 mal, grösser ist als die Löslichkeit der Salze mit 2,2'-Dihydroxy-5,5'-methylendi-(p-toluolsulfonsäure). Unter diesen bevorzugten Basen kommt Tetramethylethylendiamin eine Vorzugsstellung zu, da sich aus dessen Salz mit 2,2'-Dihydroxy-5,5'-methylendi-(p-Toluolsulfonsäure) besonders vorteilhaft die Säure freisetzen lässt.

Die schwerlöslichen Salze der 2,2'Dihydroxy-5,5'-methylendi-(p-toluolsulfonsäure) sind wichtige Zwischenprodukte bei der wirtschaftlichen Reindarstellung von 2,2'-Dihydroxy-5,5'-methylendi-(p-toluolsulfonsäure).

Ein weiterer Gegenstand der Erfindung sind daher schwerlösliche Salze der 2,2'-Dihydroxy-5,5'-methylendi-(p-toluolsulfonsäure) mit Erdalkalimetallionen, vorzugsweise Bariumionen, Erdmetallionen und Aminen, insbesondere Diaminen.

Bevorzugt sind Salze der 2,2'-Dihydroxy-5,5'-methylendi-(p-toluolsulfonsäure) mit Diaminen, in denen die Amingruppen durch eine, zwei oder drei $C_2$- bis $C_4$-Alkylenketten, insbesondere Ethylenketten verbunden sind. Besonders bevorzugt sind Salze der 2,2'-Dihydroxy-5,5'-methylendi-(p-toluolsulfonsäure) mit Piperazin, 1,4-Diazabicyclo-[2.2.2]oktan, 1,4-Di-$C_1$ bis $C_4$-alkyl-, insbesondere 1,4-Dimethylpiperazin, 1-Benzyl-4-methylpiperazin, Ethylendiamin, Di- oder Tetra-$C_1$ bis $C_4$-alkyl-, insbesondere Tetramethylethylendiamin. Ganz besonders bevorzugt ist das Tetramethylethylendiaminsalz der 2,2'-Dihydroxy-5,5'-methylendi-(p-toluolsulfonsäure).

Ein weiterer Gegenstand der Erfindung ist ein Verfahren zur Herstellung der schwerlöslichen Salze der 2,2'-Dihydroxy-5,5'-methylendi-(p-toluolsulfonsäure), das dadurch gekennzeichnet ist, dass

a) 2,2'-Dihydroxy-5,5'-methylendi-(p-toluolsulfonsäure) oder ein leicht lösliches Salz, vorzugsweise Natrium- oder Ammoniumsalz, in an sich bekannter Weise mit der entsprechenden Base umgesetzt wird, oder

b) m-Kresol-6-sulfonsäure oder deren Ammonium- oder Alkalisalz in Gegenwart von organischen Aminen oder ein Salz von m-Kresol-6-sulfonsäure mit organischen Aminen in saurem Medium mit Formaldehyd und/oder Formaldehyd abspaltenden Mitteln umgesetzt wird.

Unter schwerlöslichen Salzen der 2,2'-Dihydroxy-5,5'-methylendi-(p-toluolsulfonsäure) im Sinne dieser Erfindung werden solche verstanden, deren Löslichkeit in Wasser bei 23 °C 3 g/100 ml, vorzugsweise 1 g/100 ml nicht übersteigt.

Die Herstellung von m-Kresol-6-sulfonsäure ist bekannt. Sie erfolgt durch Sulfonierung von m-Kresol mit üblichen Sulfonierungsmitteln, wobei Gemische von m-Kresol-4-sulfonsäure und m-Kresol-6-sulfonsäure entstehen (R.D. Haworth, A. Lapworth, J. Chem. Soc. (London) 1924, 1299). Eine Isolierung der m-Kresol-6-sulfonsäure aus diesen Gemischen kann nach der zitierten Literaturstelle über ihr Bariumsalz erfolgen.

Diese Abtrennung über das Bariumsalz ist aus wirtschaftlichen Gründen bei der Herstellung in

technischem Massstab nicht günstig. Es wurde nun gefunden, dass die für die Herstellung der therapeutisch sehr wertvollen neuen Verbindung 2,2'-Dihydroxy-5,5'-methylendi-(p-toluolsulfonsäure) als Ausgangsprodukt wichtige m-Kresol-6-sulfonsäure sich überraschenderweise sehr vorteilhaft über die neue Verbindung m-Kresol-6-ammoniumsulfonat aus den bei der Sulfonierung von m-Kresol entstehenden Gemischen von m-Kresolsulfonsäuren isolieren lässt. Damit ist es möglich, den unerwünschten Umgang mit Bariumsalzen zu vermeiden. m-Kresol-6-ammoniumsulfonat ist damit ein wichtiges Zwischenprodukt zur Herstellung von 2,2'-Dihydroxy-5,5'-methylendi-(p-toluolsulfonsäure).

Die Darstellung von m-Kresol-ammoniumsulfonat erfolgt dadurch, dass dem bei der Sulfonierung von m-Kresol entstehenden Reaktionsgemisch, z.B. dem bei der Umsetzung von m-Kresol mit konzentrierter Schwefelsäure entstehenden Reaktionsgemisch, Ammoniak in zur Neutralisation der im Reaktionsgemisch vorhandenen Säuren ausreichender Menge zusetzt. Zur Neutralisation des Sulfonierungsgemisches können gasförmiges Ammoniak eingeleitet oder wässrige Ammoniaklösung zugegeben werden. Die Neutralisation mit konzentrierter wässriger Ammoniaklösung ist bevorzugt. Die Zugabe von Ammoniak erfolgt zweckmässig so, dass die Temperatur des Reaktionsgemisches nicht über 80 bis 100 °C ansteigt. Gewünschtenfalls kann die entstehende Neutralisationswärme durch Kühlung, beispielsweise durch Wasserkühlung oder durch Eiskühlung, abgeführt werden. In einer Ausführungsform des Verfahrens wird vor der Neutralisation nicht umgesetztes m-Kresol durch geeignete, mit Wasser nicht mischbare Lösungsmittel, wie beispielsweise Chloroform, extrahiert. Besonders schonend erfolgt die Neutralisation durch langsame Zugabe von Ammoniaklösung unter Kühlung, wobei die Temperatur des Reaktionsgemisches etwa zwischen 0 °C und 30 °C, vorzugsweise bei 0 °C bis 10 °C gehalten wird. Das bei der Neutralisation ausgefallene m-Kresol-6-ammoniumsulfonat wird durch geeignete Trennungsoperationen, wie beispielsweise Zentrifugieren, Filtrieren oder Dekantieren, vorzugsweise Filtrieren, aus dem Reaktionsgemisch gewonnen. Das erhaltene m-Kresol-6-ammoniumsulfonat kann noch durch Waschen mit geeigneten Lösungsmitteln, wie beispielsweise Alkanolen, insbesondere Isopropanol, von anhaftenden Resten des Reaktionsgemisches befreit werden. Nach anschliessender Trocknung, z.B. im Vakuumtrockenschrank, erhält man ein sehr reines Produkt.

m-Kresol-6-ammoniumsulfonat kann durch Umsetzung in an sich bekannter Weise mit starken Säuren oder mit saurem Ionenaustauscher in m-Kresol-6-sulfonsäure übergeführt werden, aus der bzw. deren Salzen nach den weiter oben angegebenen Verfahrensmassnahmen 2,2'-Dihydroxy-5,5'-methylendi-(p-toluolsulfonsäure) und deren Salze hergestellt werden können. Es ist aber auch möglich, nach den oben angegebenen Verfahrensmassnahmen m-Kresol-6-ammoniumsulfonat durch Umsetzung mit Formaldehyd direkt in 2,2'-Dihydroxy-5,5'-methylendi-(p-toluolsulfonsäure) und deren Salze überzuführen. Die Freisetzung der m-Kresol-6-sulfonsäure aus m-Kresol-6-ammoniumsulfonat mit Hilfe von sauren Ionenaustauscherharzen erfolgt entweder in einer Austauschsäule oder durch direktes Zusammengeben einer Lösung des m-Kresol-6-ammoniumsulfonates und des Ionenaustauscherharzes. Der Ionenaustausch wird vorzugsweise in wässriger Lösung bei Umgebungstemperatur oder Temperaturen bis etwa 60 °C durchgeführt. Jedoch führt auch die Verwendung von anderen genügend polaren Lösungsmitteln, wie z.B. Alkanolen, vorzugsweise Isopropanol, zu guten Ergebnissen.

Die Freisetzung der 2,2'-Dihydroxy-5,5'-methylendi-(p-toluolsulfonsäure) aus ihren schwerlöslichen Salzen erfolgt in an sich bekannter Weise durch Umsetzung mit starken Säuren. Bevorzugt wird die Freisetzung der 2,2'-Dihydroxy-5,5'-methylendi-(p-toluolsulfonsäure) aus ihren Salzen mit Hilfe von sauren Ionenaustauschern. Es ist dabei möglich, die schwerlöslichen Salze direkt mit dem sauren Ionenaustauscher in Kontakt zu bringen oder die schwerlöslichen Salze vorher durch Umsetzung mit Alkalilauge, vorzugsweise Natronlauge, in ein leichter lösliches Alkali-, vorzugsweise Natriumsalz umzuwandeln. Die Freisetzung der 2,2'-Dihydroxy-5,5'-methylendi-(p-toluolsulfonsäure) aus ihren Salzen mit Hilfe von sauren Ionenaustauscherharzen erfolgt entweder in einer Austauschsäule oder durch direktes Zusammengeben einer Lösung eines Salzes der 2,2'-Dihydroxy-5,5'-methylendi-(p-toluolsulfonsäure) und des Ionenaustauschers. Der Ionenaustausch wird vorzugsweise in wässriger Lösung durchgeführt. Jedoch führt auch die Verwendung von anderen genügend polaren Lösungsmitteln, wie z.B. Alkanolen, vorzugsweise Isopropanol zu guten Ergebnissen. Die bei der Freisetzung der erfindungsgemässen Säure mit Hilfe von Ionenaustauschern zu wählende Temperatur richtet sich nach der Löslichkeit des jeweiligen Salzes in dem verwendeten Lösungsmittel. Bei Vorliegen von leicht löslichen Salzen, wie beispielsweise in Wasser gut löslichem Natriumsalz, wird vorzugsweise bei der Umgebungstemperatur gearbeitet. Bei schwerer löslichen Salzen wird bei 40 bis 90 °C, vorzugsweise 80 bis 90 °C, gearbeitet. Eine bevorzugte Ausgestaltung des erfindungsgemässen Verfahrens besteht darin, als Zwischenprodukte erhaltene schwerlösliche Salze der 2,2'-Dihydroxy-5,5'-methylendi-(p-toluolsulfonsäure) durch Umsetzung mit einer Alkalilauge, vorzugsweise Natronlauge, in das entsprechende Alkalisalz umzuwandeln und aus diesem mit Hilfe eines sauren Ionenaustauschers die Säure freizusetzen.

Die Verbindung 2,2'-Dihydroxy-5,5'-methylendi-(p-toluolsulfonsäure) und ihre Salze mit organischen und anorganischen Salzen wirken adstringierend, bakterizid, trichomonazid, fungizid und hämostyptisch. Sie besitzen eine selektive Wirkung auf abgestorbenes sowie krankhaft verändertes Gewebe, das koaguliert und abgestossen

wird, während gesundes Gewebe nicht beeinträchtigt wird.

Ihre Toxizität ist geringer als die des aus einem Gemisch von Kondensationsprodukten aus m-Kresolsulfonsäuren und Formaldehyd bestehenden Handelsprodukts. In der nachstehenden Tabelle sind die bei intravenöser Verabreichung an Maus und Ratte ermittelten $LD_{50}$-Werte des Dinatriumsalzes der 2,2'-Dihydroxy-5,5'-methylendi-(p-toluolsulfonsäure) (A) und des mit Natriumhydroxid neutralisierten Handelsprodukts (B) aufgeführt.

Tabelle

| | $LD_{50}$ (mg/kg« | |
|---|---|---|
| Tierart | A | B |
| Maus ♂ | 1100 | 380 |
| Maus ♀ | 1050 | 340 |
| Ratte ♂ | 930 | 390 |
| Ratte ♀ | 990 | 420 |

Ein weiterer Gegenstand der Erfindung sind daher Arzneimittel enthaltend 2,2'-Dihydroxy-5,5'-methylendi-(p-toluolsulfonsäure) und deren Salze mit pharmakologisch verträglichen anorganischen und organischen Basen.

Die erfindungsgemässen Arzneimittel werden in der Frauenheilkunde vor allem zur Behandlung von Vaginitis, Fluor vaginalis und zervikalis, Portioerosion und -ektopie, zur Blutstillung nach Probeexcisionen und Entferung von Zervixpolypen, Druckgeschwüren bei Pessarträgerinnen und zur Nachbehandlung bei Elektrokoagulation eingesetzt. In der Urologie eignen sich die erfindungsgemässen Arzneimittel beispielsweise zur Behandlung von Zystitis, bakterieller und Trichomonadeninfektion, Erosionen und Papillomen, sowie von Urethritis. In der Chirurgie lassen sich die erfindungsgemässen Arzneimittel zur Stillung kapillärer Blutungen und zur Abstossung nekrotischen Gewebes nach Verbrennungen und in der Wundbehandlung einsetzen. In der Dermatologie können unter anderem Verbrennungen dritten Grades, Dekubitusdefekte, Ulcera cruris, Aphthen, Urethralerosionen und -polypen, eröffnete Furunkel, Karbunkel und Panaritien behandelt werden. In der Hals-, Nasen-, Ohrenheilkunde können die erfindungsgemässen Arzneimittel insbesondere zur Behandlung von Gewebsnekrosen in Radikalhöhlen, Otitis externa. Stomatitis aphthosa, Rhagaden, Nasenbluten, Pharyngitis und zur Blutstillung nach Tonsillektomie eingesetzt werden. In der Proktologie können die erfindungsgemässen Arzneimittel zur Behandlung von äusseren und inneren Hämorrhoiden, insbesondere mit entzündlichen Begleiterscheinungen und Blutungen, Analfissuren, Analrhagaden, Analekzemen, Analpruritus und zur Wundbehandlung nach Operationen eingesetzt werden. Die erfindungsgemässen Arzneimittel können ganz allgemein zur Beschleunigung des Heilungsprozesses von Wunden verschiedensten Ursprungs verwendet werden.

Gegenstand der Erfindung ist daher auch die 2,2'-Dihydroxy-5,5'-methylendi-(p-toluolsulfonsäure) und deren pharmakologisch verträgliche Salze mit organischen und anorganischen Basen zur Anwendung bei der Behandlung von krankem Gewebe.

Die erfindungsgemässen Arzneimittel können in verschiedenen Zubereitungsformen vorliegen. Der Wirkstoffgehalt dieser Arzneiformen beträgt in der Regel 0,5 bis 95, vorzugsweise 1 bis 85 Gew.-%, bezogen auf das fertige Arzneimittel.

Da das neue Arzneimittel bevorzugt lokal angewendet wird, sind Lösungen, Vaginalkugeln, Gele, Salben, Suppositorien, Tabletten zur lokalen Anwendung oder Konzentrate besonders bevorzugte Zubereitungsformen.

Die pharmazeutischen Zubereitungen gemäss der Erfindung enthalten, wenn sie in Form einer Einheitsdosis vorliegen, z.B. als Vaginaltablette, als Vaginalkugel, als Suppositorium 10 bis 150, insbesondere 20 bis 100 mg des Wirkstoffes.

Unter «Einheitsdosis» im Sinne der vorliegenden Erfindung wird eine physikalisch bestimmte Einheit, die eine individuelle Menge des aktiven Bestandteils in Mischung mit einem pharmazeutischen Verdünnungsmittel dafür oder zusammen mit einem pharmazeutischen Trägerstoff enthält, verstanden. Dabei wird die Menge des Wirkstoffes so gewählt, dass eine oder mehrere Einheiten üblicherweise für eine einzelne therapeutische Verabreichung benötigt werden.

Es kann die Einheitsdosis aber auch unterteilbar sein, z.B. bei mit Kerben versehenen Tabletten, wenn für eine einzelne therapeutische Verabreichung nur ein Bruchteil, wie eine Hälfte oder ein Viertel, der unterteilbaren Einheit benötigt wird.

Vaginalkugeln haben in der Regel den selben Wirkstoffgehalt je Kugel wie Tabletten. Gele und Salben enthalten in der Regel 5 bis 30 mg des Wirkstoffes je Gramm.

Wirkstofflösungen enthalten in der Regel 0,5 bis 10, insbesondere 1 bis 5 Gew.-% an Wirkstoff.

Wirkstoffkonzentrate enthalten in der Regel 10 bis 40, insbesondere 20 bis 40 Gew.-% an Wirkstoff.

Neben den neuen Wirkstoffen können die pharmazeutischen Zubereitungen beispielsweise einen oder mehrere pharmakologisch aktive Bestandteile aus anderen Arzneimittelgruppen enthalten, beispielsweise Sulfonamide, z.B. Sulfathiazol, Sulfacetamid; Antibiotica, z.B. Neomycin, Bacitracin, Chloramphenicol, Tetracyclin; Antiseptica, z.B. 9-Aminoacridin; trichomonazide Wirkstoffe, z.B. Metronidazol; Mykostatica, z.B. Nystatin; Antiphlogistica, z.B. Hydrocortison oder Östrogene, z.B. Diethylstilböstrol, Ethinylöstradiol oder Mestranol. Soweit es sich bei den vorstehend genannten aktiven Bestandteilen aus anderen Arzneimittelgruppen um solche handelt, die mit 2,2'-Dihydroxy-5,5'-methylendi-(p-toluolsulfonsäure) und/oder deren Salzen chemisch unverträglich sind, z.B. aufgrund von Säureempfindlichkeit, erfolgt auf an sich bekannte Weise eine galenische Separierung der unverträglichen aktiven Bestandteile innerhalb der gewünschten Zubereitungsform. Beispielsweise werden die unverträglichen Bestandteile in verschiedene Schichten einer Schichttablette oder in verschiedene in eine

Kapsel abgefüllte Pellets oder in verschiedene Schichten von Mehrschicht-Suppositorien oder Vaginalkugeln eingearbeitet.

Die verschiedenen Zubereitungsformen ermöglichen eine vielseitige Anwendungsweise. Konzentrate und Lösungen werden zur Tuschierungsbehandlung, auch in der Hals-, Nasen-, Ohrenheilkunde und bei zahnärztlicher Behandlung angewandt.

Es ist auch möglich, eine kombinierte Anwendung verschiedener Zubereitungsformen der erfindungsgemässen Arzneimittel durchzuführen. Beispielsweise wird alle ein, zwei oder drei Tage eine Behandlung des erkrankten Körperteils mit einer 30 bis 40%igen, vorzugsweise 36%igen Lösung der 2,2'-Dihydroxy-5,5'-methylendi-(p-toluolsulfonsäure) und/oder ihrer Salze durch Auftragen, Spülen oder Baden durchgeführt. Zwischen diesen Behandlungen werden ein- bis zweimal täglich Gel oder Salbe bzw. Tabletten zur lokalen Behandlung, Vaginalkugeln oder Suppositorien angewendet.

Gele und Salben werden meist mit einem Ansatzrohr in die Vagina eingeführt. Scheidenspülungen können mittels einer Vaginaldusche vorgenommen werden. Vaginalkugeln oder Vaginaltabletten werden meist abends im Liegen in die Scheide eingeführt.

Die nachfolgenden Beispiele erläutern die Erfindung näher, ohne sie einzuschränken. Die angeführten Temperaturen sind in °C angegeben. «F.» bedeutet Schmelzpunkt.

Beispiele
(In den Beispielen 14 bis 25 wird die Herstellung von Ausgangsprodukten beschrieben)

Beispiel 1
2,2'-Dihydroxy-5,5'-methylendi-(p-toluolsulfonsäure)

a) Die Mischung von 100 g (0,446 mol) m-Kresol-6-sulfonsäure-dihydrat in 300 ml Wasser und 28 ml (0,36 mol) Formalinlösung (37%ig) wird 5 Tage bei 20–22 °C gerührt und anschliessend im Vakuum eingedampft. Der ölige Rückstand wird mit 100 ml Acetonitril verrührt und abgesaugt. Man erhält die 2,2'-Dihydroxy-5,5'-methylendi-(p-toluolsulfonsäure) als Trihydrat.

Ausbeute: 43 g (42% d.Th.)

Der Schmelzpunkt variiert entsprechend dem Wassergehalt und liegt zwischen 132 °C (4 $H_2O$) und 142 °C (wasserfrei).

b) Eine Suspension von 10 g (44,6 (44,6 mmol) m-Kresol-6-sulfonsäure-dihydrat und 1 g Paraformaldehyd in Wasser wird 8 Tage bei 20–22 °C gerührt. Die klare Lösung wird wie unter a) beschrieben aufgearbeitet.

Ausbeute: 4,6 g (45% d.Th.)

Aus der Mutterlauge können durch säulenchromatographische Reinigung weitere Mengen der Titelverbindung erhalten werden.

c) 500 g Piperazin-[2,2'-dihydroxy-5,5'-methylendi-(p-toluolsulfonat)] werden in 500 ml 50%iger Natronlauge bei 60 ° gelöst. Bei gleicher Temperatur werden 3 l Methanol zugegeben und das ausgefallene Natriumsalz abfiltriert, mit heissem Methanol gewaschen und im Vakuum getrocknet. 490 g dieses Natriumsalzes werden unter Erwärmen in 600 ml Wasser gelöst, auf Raumtemperatur gekühlt und über eine Ionenaustauschersäule gegeben (Säulenmasse 200 × 10 cm; 2,5 kg Amberlite® IR 120). Das Eluat wird solange gesammelt, bis ein pH-Wert von 3 bis 4 erreicht ist; die erhaltene Lösung wird im Vakuum bei 40 °C zur Trockne eingedampft und der Rückstand aus Acetonitril umkristallisiert.

d) Eine Sephadex®-Säule K 100/100 (Fa. Pharmazia), 7 l Inhalt, mit Heizmantel, wird mit 5 kg Ionenaustauscher Amberlite® IR 120 stark sauer beschickt und mit Hilfe eines Thermostaten auf 85 °C erwärmt. Kontinuierlich wird mittels Schlauchpumpe eine bei 85 °C gesättigte wässrige Lösung von insgesamt 2 kg (3,127 mol) Tetramethylethylendiammonium-[2,2'-dihydroxy-5,5'-methylendi-(p-toluolsulfonat)] aufgegeben. Das saure Eluat wird zunächst im Dünnschichtverdampfer und anschliessend im Rotationsverdampfer bis zur Trockne eingedampft.

Ausbeute: 1360 g (95% d.Th.); F. 135–140 °C.

e) 4 l Ionenaustauscher Amberlite® IR 120 (saure Form, mit Isopropanol gewaschen) werden in 1,6 lt Isopropanol mit 430 g Dinatriumsalz des 2,2'-Dihydroxy-5,5'-methylendi-(p-toluolsulfonsäure)-monohydrats 6 Stunden bei 60–80 °C gerührt. Nach Abkühlung wird vom Ionenaustauscher abfiltriert und das Filtrat im Vakuum eingeengt, zuletzt nach Zusatz von 500 ml Eisessig. Der Destillationsrückstand wird mit 800 ml Dichlormethan verrührt, wobei Kristallisation stattfindet.

Ausbeute: 330 g (78% d.Th.) 2,2'-Dihydroxy-5,5'-methylendi-(p-toluolsulfonsäure)-trihydrat; F. 135–138 °C.

Beispiel 2
Piperazin-[2,2'-Dihydroxy-5,5'-methylendi-(p-toluolsulfonat)]

a) 100 g (0,486 mol) m-Kresol-6-ammoniumsulfonat werden in 325 ml 6N Schwefelsäure suspendiert und unter Rühren auf 40 °C Innentemperatur erwärmt. Nach Zugabe von 35 ml 37%iger Formalinlösung (0,453 mol) wird 3½ Stunden bei gleicher Temperatur gerührt. Dann wird im Eisbad gekühlt und nicht umgesetztes Ausgangsprodukt abgesaugt. Das Filtrat wird noch in der Kälte mit 18 g Piperazin versetzt und das alsbald ausgeschiedene kristalline Produkt nach einigen Stunden Stehen bei Raumtemperatur abgesaugt. Der Rückstand wird mit Wasser, Ethanol und eventuell Ether gewaschen und im Vakuum bei 50–100 °C getrocknet.

Ausbeute: 53 g (55% d.Th.); F. > 380 °C.

b) Das Gemisch aus 5 g (22 mmol) m-Kresol-6-sulfonsäure-dihydrat und 5 ml Formaldehydlösung (37%ig, 62 mmol) wird 4 Stunden bei 20 bis 22 °C gerührt und dann mit 1 g Piperazin in 15 ml Wasser versetzt. Der ausgefallene Niederschlag wird abgesaugt und mit Wasser und Ethanol gewaschen.

Ausbeute: 3,5 g (66% d.Th.)

c) 2 g (4,3 mmol) 2,2'-Dihydroxy-5,5'-methylen-

di-(p-toluolsulfonsäure) werden in 10 ml Wasser mit 0,4g (4,7 mmol) wasserfreiem Piperazin 15 Minuten gerührt. Der kristalline Niederschlag wird abgesaugt.

Ausbeute: 2 g (97% d.Th.).

Beispiel 3

Tetramethylethylendiammonium-[2,2'-dihydroxy-5,5'-methylendi-(p-toluolsulfonat)]-monohydrat

a) 1000 g (4,06 mol) Tetramethylethylendiammonium-bis-(m-kresol-6-sulfonat) werden in einem Gemisch aus 500 ml Wasser und 212 g (ca. 2,08 mol) konz. Schwefelsäure suspendiert. In die auf 30 °C gekühlte Suspension werden 288 g (266 ml, 3,56 mol) Formalin (37%ig) gegeben und 48 Stunden bei 30 °C gerührt. Dann wird die mit Eiswasser gekühlte Suspension abgesaugt, neutralgewaschen und im Vakuum getrocknet.

Ausbeute: 730 g )69% d.Th.).

Das Produkt kann aus 3 l Wasser umkristallisiert werden.

F. 228–232 °C

b) Eine auf 80 °C erhitzte Mischung aus 51 g (0,248 mol) m-Kresol-6-ammoniumsulfonat, 16,9 g (0,145 mol) Tetramethylethylendiamin und 38,6 g (21 ml, 0,39 mol) konz. Schwefelsäure in 50 ml Wasser wird mit 20 ml einer 36%igen Formaldehydlösung versetzt und 6 Tage bei 20 bis 23 °C gerührt. Die entstandene kristalline Fällung wird abgesaugt und mit Wasser und Methanol gewaschen.

Ausbeute: 40 g (62% d.Th.).

Beispiel 4

1,4-Dimethylpiperazin-[2,2'-dihydroxy-5,5'-methylendi-(p-toluolsulfonat)]

a) Eine Mischung aus 10 g (48,7 mmol) m-Kresol-6-ammoniumsulfonat, 1,3 ml (23,6 mmol) Schwefelsäure (d = 1,84) und 3,5 ml (45,3 mmol) Formaldehydlösung (37%ig) wird 30 Minuten bei 60 °C gerührt. Dann werden bei gleicher Temperatur im Verlauf von weiteren 3 Stunden 1,95 ml (14,5 mmol) 1,4-Dimethylpiperazin in Portionen von 0,1 ml zugegeben. Nach Erkalten wird der Niederschlag isoliert.

Ausbeute: 6,3 g (52% d.Th.); F. 286–289 °C.

b) In ein Gemisch aus 10 g (38 mmol) 1,4-Dimethylpiperazin-(m-kresol-6-sulfonat) und 3,8 g konz. Schwefelsäure in 10 ml Wasser werden bei 45 °C 2,7 ml (35,3 mmol) Formaldehydlösung (36%ig) gegeben, 12 Stunden bei gleicher Temperatur gerührt und der Niederschlag abfiltriert.

Ausbeute: 6,1 g (64% d.Th.)

c) In die Lösung von 7,8 g (0,038 mol) m-Kresol-6-ammoniumsulfonat in 10 ml Wasser werden 3,15 ml konz. Schwefelsäure und 2,17 g (0,019 mol) 1,4-Dimethylpiperazin gegeben. Nach etwa 15 Minuten werden 2,7 ml 36%ige Formaldehydlösung zugefügt und 22 Stunden bei 50 °C gerührt. Der Niederschlag wird abgesaugt und im Vakuum getrocknet.

Ausbeute: 5,4 g (57% d.Th.).

d) Ein Gemisch aus 3 g (13,4 mmol) m-Kresol-6-sulfonsäure-dihydrat und 0,32 g (2,2 mmol) Hexamethylentetramin in 10 ml Wasser wird 48 Stunden bei 45 °C gerührt. Dann werden 0,76 g (6,2 mmol) 1,4-Dimethylpiperazin in 10 ml Wasser zugegeben und mit verdünnter Salzsäure auf pH 1 gestellt. Nach 1 Stunde Rühren wird der Niederschlag abgesaugt, mit Wasser und Methanol gewaschen und im Vakuum getrocknet.

Ausbeute: 1,2 g (36% d.Th.)

e) Die Mischung aus 3,6 g (90 mmol) Natriumhydroxid, 10 g (44,6 mmol) m-Kresol-6-sulfonsäure-dihydrat, 3,4 ml (44,3 mmol) 36%ige Formaldehydlösung in 20 ml Wasser wird 30 Stunden bei 22 bis 25 °C gerührt. Man neutralisiert die Lösung mit verdünnter Salzsäure und dampft im Vakuum zur Trockne ein. Es werden 6,3 g Rückstand erhalten, der, aus Ethanol/Wasser umgefällt, sich ab 270 °C zersetzt.

2 g dieses Produktes (Natriumsalz der 4-Hydroxymethylen-m-kresol-6-sulfonsäure) werden mit 2 g m-Kresol-6-sulfonsäure-dihydrat in 20 ml Wasser 8 Stunden auf 90 °C erhitzt. Dann werden 1,1 ml 1,4-Dimethylpiperazin zugesetzt und auf pH 4 eingestellt. Der Niederschlag wird abgesaugt, mit Wasser gewaschen und getrocknet.

Ausbeute: 1,2 g (30% d.Th.).

Beispiel 5

1,4-Diazabicyclo[2.2.2]oktan-[2,2'-dihydroxy-5,5'-methylendi-(p-toluolsulfonat)]

a) Die Mischung aus 10,2 g (50 mmol) m-Kresol-6-ammoniumsulfonat, 40 ml Wasser, 5,6 ml konz. Schwefelsäure, 2,8 ml (25 mmol) 1,4-Diazabicyclo-[2.2.2]oktan und 4 ml 36%ige Formaldehydlösung wird 4 Stunden bei 45 °C gerührt. Nach Erkalten wird der Niederschlag abgesaugt, mit Wasser und Methanol gewaschen und getrocknet.

Ausbeute: 8,4 g (67% d.Th.) des Salzes aus 1 mol 2,2'-Dihydroxy-5,5'-methylendi-(p-toluolsulfonsäure) mit 1 mol 1,4-Diazabicyclo[2.2.2]oktan. F. > 300 °C.

b) Die Lösungen aus 4,6 g (10 mmol) 2,2'-Dihydroxy-5,5'-methylendi-(p-toluolsulfonsäure)-tetrahydrat in 30 ml Wasser und 1,1 g (10 mmol) 1,4-Diazabicyclo[2.2.2]oktan in 20 ml Wasser werden unter Rühren vereinigt. Der Niederschlag wird abgesaugt, mit Wasser gewaschen und getrocknet.

Ausbeute: 4,9 g (98% d.Th.).

Beispiel 6

Dinatrium-2,2'-dihydroxy-5,5'-methylendi-(p-toluolsulfonat)-monohydrat

In die 60 °C warme Lösung von 220 g (5,5 mol) Natriumhydoxid in 800 ml Wasser werden unter Stickstoffbegasung 522,6 g (1 mol) Tetramethylethylendiammonium- [2,2' dihydroxy-5,5'-methylendi-(p-toluolsulfonat)]-monohydrat eingetragen und die freigesetzte organische Base abdestilliert. Der viskose Rückstand wird in eine kräftig gerührte Mischung von 240 ml Eisessig und 800 ml Methanol eingegossen. Der feinkristalline Niederschlag wird abgesaugt, mit Methanol gewaschen und im Vakuum getrocknet.

Ausbeute: 431 g (96% d.Th.); F. ca. 290 °C (Zersetzung).

Beispiel 7

1,2-Ethylendiammonium-[2,2'-dihydroxy-5,5'-methylendi-(p-toluolsulfonat)]

5,8 g (12,9 mmol) 2,2'-Dihydroxy-5,5'-methylendi-(p-toluolsulfonsäure)-tetrahydrat und 0,78 g (12,9 mmol) 1,2-Diaminoethan werden in 20 ml Wasser gemischt und der kristalline Niederschlag abfiltriert.

Ausbeute: 5,2 g (90% d.Th.); F. > 270 °C.

Beispiel 8

1-Methylpiperazin-[2,2'-Dihydroxy-5,5'-methylendi-(p-toluolsulfonat)]

3 g (6,5 mmol) 2,2'-Dihydroxy-5,5'-methylendi-(p-toluolsulfonsäure)-tetrahydrat werden in 15 ml eiskaltem Methanol mit 0,7 ml 1-Methylpiperazin versetzt und der Niederschlag abgesaugt.

Ausbeute: 3 g (95% d.Th.); F. 318 °C (Zersetzung).

Beispiel 9

1,4-Dibenzylpiperazin-[2,2'-Dihydroxy-5,5'-methylendi-(p-toluolsulfonat)]

Die Lösungen aus 4,6 g (10 mmol) 2,2'-Dihydroxy-5,5'-methylendi-(p-toluolsulfonsäure)-tetrahydrat in 100 ml Wasser und 2,7 g (10 mmol) 1,4-Dibenzylpiperazin in 30 ml 2N Schwefelsäure werden vereinigt. Der Niederschlag wird abgesaugt, mit Wasser und Methanol gewaschen und getrocknet.

Ausbeute: 6,5 g (98% d.Th); F. 260 °C (Zersetzung).

Beispiel 10

1-Methyl-4-benzylpiperazin-[2,2'-dihydroxy-5,5'-methylendi-(p-toluolsulfonat)]

Analog Beispiel 9 wird mit 1-Methyl-4-benzylpiperazin die Titelverbindung in 83%iger Ausbeute erhalten. F. ca. 190 °C (Zersetzung).

Beispiel 11

Di-morpholin-[2,2'-dihydroxy-5,5'-methylendi-(p-toluolsulfonat)]

Zu einer Lösung von 3 g (6,5 mmol) 2,2'-Dihydroxy-5,5'-methylendi-(p-toluolsulfonsäure)-tetrahydrat in 15 ml Methanol werden 1,22 ml (14 mmol) Morpholin gegeben. Der entstandene Niederschlag abgesaugt und im Vakuum getrocknet.

Ausbeute: 3,6 g (98,5% d.Th.); F. 205–206 °C.

Beispiel 12

Barium-[2,2'-dihydroxy-5,5'-methylendi-(p-toluolsulfonat)]

Zu einer Lösung von 11,5 g (25 mmol) 2,2'-Dihydroxy-5,5'-methylendi-(p-toluolsulfonsäure)-tetrahydrat in 50 ml Wasser wird eine Lösung von 6,1 g (25 mmol) Bariumchlorid-dihydrat in 20 ml Wasser gegeben. Der Niederschlag wird abfiltriert, mit Wasser und Aceton gewaschen und getrocknet.

Ausbeute: 12 g (92% d.Th.); F. ab 196 °C Zersetzung.

Beispiel 13

2,2'-Dihydroxy-5,5'-methylendi-(p-toluolsulfonsäure)-diharnstoffsalz

Die Lösungen aus 100 g (0,217 mol) 2,2'-Dihydroxy-5,5'-methylendi-(p-toluolsulfonsäure)-Tetrahydrat und 26 g (0,434 mol) Harnstoff in je 150 ml Methanol werden vermischt und eine halbe Stunde gerührt. Nach Kühlung auf 0 °C wird der Niederschlag abgesaugt und das Filtrat zur Gewinnung weiterer Substanz eingeengt.

Ausbeute: 95 g (86% d.Th.) F. ca. 200 °C (Zersetzung).

Beispiel 14

m-Kresol-ammoniumsulfonat

Innerhalb von etwa 5 Minuten werden zu 7,5 g (69,4 mmol) m-Kresol bei 22 °C unter Rühren 7,08 g (69,4 mmol) konzentrierte Schwefelsäure (D=1,84) getropft. Danach wird das Reaktionsgemisch auf 50 °C erwärmt und 3 Stunden bei dieser Temperatur gerührt. Anschliessend wird auf Raumtemperatur abgekühlt, mit 20 ml Wasser verdünnt und zur Entfernung von nicht umgesetztem m-Kresol 6 mal mit je 20 ml Chloroform extrahiert.

Die wässrige Phase wird auf 0 °C gekühlt, mit 8 ml 25%iger Ammoniaklösung versetzt und 1 Stunde bei 0 °C nachgerührt. Das ausgefallene m-Kresol-6-ammoniumsulfonat wird abgesaugt und mit Isopropanol nachgewaschen. Der Niederschlag ist dünnschichtchromatographisch rein und wird im Vakuumtrockenschrank bei 60 °C getrocknet.

Ausbeute: 4,2 g (30% d.Th.); F. 255–257 °C (Zersetzung).

Beispiel 15

m-Kresol-6-sulfonsäure

100 g (0,488 mol) m-Kresol-6-ammoniumsulfonat werden in 200 ml Wasser unter Erwärmen auf 90 °C gelöst und auf eine auf 90 °C gehaltene Ionenaustauschersäule mit 300 g feuchtem Amberlite IT 120 (saure Form) gegeben. Durch Eindampfen des wässrigen Eluates bei 50 °C im Vakuum und Umkristallisieren des Rückstandes aus Acetonitril erhält man die m-Kresol-6-sulfonsäure als Dihydrat.

Ausbeute: 104 g (95% d.Th.); F. 90–93 °C.

Beispiel 16

m-Kresol-6-tetramethylethylendiammoniumsulfonat

a) 2000 g (9,63 mol) m-Kresol-6-ammoniumsulfonat werden portionsweise unter starkem Rühren in eine 95 °C heisse Lösung von 700 g (17,5 mol) Natriumhydroxid in 3 l Wasser gegeben. Zur vollständigen Entfernung des freigesetzten Ammoniaks wird weitere 2 Stunden unter Einleiten von Stickstoff gekocht. Diese Lösung wird in ein Gemisch aus 820 g (9,4 mol) konzentrierter Schwefelsäure in 6 l Wasser gegeben. Das ausgefallene Kristallisat geht bei der anschliessenden Zugabe von 560 g (4,82 mol) 1,2-Bis-(dimethylamino)-ethan in der Hitze wieder in Lösung. Nach Abkühlen wird das ausgefallene Salz abgesaugt, gewaschen und getrocknet. Es enthält 0,5 mol Base auf 1 mol m-Kresol-6-sulfonsäure.

Ausbeute: 2044 g (86% d.Th.); F. 183–185 °C.

b) das gleiche Produkt wird in praktisch quantitativer Ausbeute erhalten, wenn man anstelle des Ammoniumsalzes die freie m-Kresol-6-sulfonsäure (Dihydrat) mit der äquivalenten Menge 1,2-Bis-(dimethylamino)-ethan in Wasser umsetzt.

c) Eine andere Arbeitsweise besteht darin, dass zur Austreibung des Ammoniaks aus dem m-Kresol-6-ammoniumsulfonat nicht Natriumhydroxid in wässriger Lösung, sondern 1,2-Bis-(dimethylamino)-ethan in Chlorbenzol verwendet wird. Man erhält z.B. aus 233 g (1.14 mol) m-Kresol-6-ammoniumsulfonat in 250 ml Chlorbenzol mit 190 ml (1,26 mol) 1,2-Bis-(diethylamino)-ethan nach Erhitzen auf ca. 160 °C (30 bis 60 Minuten), Filtration und Versetzen des Filtrats mit 75 ml Eisessig 242 g (86% d.Th.) der Titelverbindung; F. 182–184 °C.

Beispiel 17

1,4-Dimethylpiperazin-(m-kresol-6-sulfonat)

a) Zur Lösung von 100 g (0,445 mol) m-Kresol-6-sulfonsäure-Dihydrat in 300 ml Wasser werden 30 ml (0,223 mol) 1,4-Dimethylpiperazin gegeben. Nach 0,5 Stunden Rühren bei 0 °C wird der Niederschlag abfiltriert, mit Wasser und Methanol gewaschen und getrocknet. Ausbeute: 109 g (93% d.Th.); F. 236–238 °C. Das Salz enthält 0,5 ml 1,4-Dimethylpiperazin pro mol m-Kresol-6-sulfonsäure und 1 mol Kristallwasser.

b) Das gleiche Salz wird erhalten, wenn als Ausgangsprodukt das m-Kresol-6-ammoniumsulfonat eingesetzt wird. Nach Zugabe äquimolarer Mengen Salzsäure und halbäquimolarer Mengen 1,4-Dimethylpiperazin fällt das Salz in praktisch quantitativer Ausbeute an.

Beispiel 18

Morpholin-m-kresol-6-sulfonat

100 g (0,49 mol) m-Kresol-6-ammoniumsulfonat werden mit 47 ml (0,54 mol) Morpholin in 200 ml Wasser 10 Stunden auf 100 °C erhitzt und die Lösung im Vakuum zur Trockne gebracht. Der Rückstand wird mit 200 ml Aceton aufgenommen, der unlösliche Rückstand abfiltriert und mit Aceton und Ether gewaschen. Ausbeute: 106 g (79% d.Th.); F. 167–170 °C.

Beispiel 19

1-Benzyl-4-methylpiperazin-m-kresol-6-sulfonat

Die Lösungen von 4,1 g (20 mmol) m-Kresol-6-ammoniumsulfonat in 45 ml Wasser und von 2,6 g (10 mmol) 1-Benzyl-4-methylpiperazin-dihydrochlorid in 15 ml Wasser werden unter Rühren vereinigt und der Niederschlag abfiltriert. Ausbeute: 3,9 g (53% d.Th.); F. 142–146 °C.

Beispiel 20

1,4-Dibenzylpiperazin-m-kresol-6-sulfonat

Auf gleiche Weise wie in Beispiel 6 beschrieben erhält man aus 5,3 g (20 mmol) Dibenzylpiperazin in 30 ml 2n Schwefelsäure gelöst und 8,2 g (40 mmol) m-Kresol-6-ammoniumsulfonat in 200 ml Wasser 11,6 g (92% d.Th.) der Titelverbindung; F. 223 °C.

Beispiel 21

1,4-Diazabicyclo[2.2.2]octan-m-kresol-6-sulfonat

Die Lösungen von 2,24 g (10 mmol) m-Kresol-6-sulfonsäure-dihydrat in 10 ml Wasser und 0,55 g (5 mmol) 1,4-Diazabicyclo[2.2.2]octan in 10 ml Wasser werden unter Rühren vereinigt und die Fällung abfiltriert. Ausbeute: 1,4 g (57% d.Th.); F. 250 °C.

Beispiel 22

Barium-m-kresol-6-sulfonat

100 g (0,48 mol) m-Kresol-6-ammoniumsulfonat werden mit 200 ml Wasser und 10 ml konzentrierter Salzsäure bis zur klaren Lösung erwärmt, dann 50 g (0,2 mol) Bariumchlorid-dihydrat, gelöst in 120 ml Wasser, zugefügt und die Ausfällung abfiltriert. Ausbeute: 77 g (75% d.Th.); F. >250 °C.

Beispiel 23

Ethylendiammonium-di-(m-kresol-6-sulfonat)

In die Lösung von 5 g (22 mmol) m-Kresol-6-sulfonsäure-Dihydrat in 20 ml Wasser werden 0,7 g (12 mmol) Ethylendiamin zugesetzt und der Niederschlag abfiltriert. Ausbeute 3,4 g (70% d.Th.); F. 226–229 °C.

Beispiel 24

Piperazin-di-(m-kresol-6-sulfonat)

Auf die gleiche Weise wie im Beispiel 23 werden aus 2 g m-Kresol-6-sulfonsäure-Dihydrat und 0,4 g Piperazin in 10 ml Wasser 1,4 g (69% d.Th.) der Titelverbindung erhalten. F. 254–256 °C (Zersetzung)

Beispiel 25

1-Methylpiperazin-m-kresol-6-sulfonat

100 g (0,49 mol) m-Kresol-6-ammoniumsulfonat werden mit 48,6 g (0,49 mol) 1-Methylpiperazin in 200 ml Wasser 7 bis 8 Stunden gekocht und dann im Vakuum zur Trockne gebracht. Der Rückstand wird mit 50 ml Aceton verrührt und der Niederschlag abgesaugt. Ausbeute: 69 g (49% d.Th.), F. 129–131 °C (aus Methanol).

Beispiel 26

Konzentrat zur Tuschierungsbehandlung

Herstellung einer Charge von 100 kg:

36 kg 2,2'-Dihydroxy-5,5'-methylendi-(p-toluolsulfonsäure) werden unter Rühren in einem $V_2A$-Behälter in 64 kg destilliertem Wasser eingetragen. Nach 1-stündigem Rühren ist die Lösung homogen und wird in Glasflaschen à 100 ml abgefüllt.

Beispiel 27

Vaginalkugeln

Herstellung einer Charge von 100 000 Vaginalkugeln à 90 mg Wirkstoff:

9 kg 2,2'-Dihydroxy-5,5'-methylendi-(p-toluolsulfonsäure) werden in 9 l destilliertem Wasser gelöst, 57 kg Polyethylenglykol 4000 und 225 kg Polyethylenglykol 1500 werden bei 70 ° geschmolzen. Unter Rühren wird in diese Schmelze die wässrige Lösung des Wirkstoffs eingetragen.

Nach Abkühlen auf 50° wird die fertige Masse in Ovula-Formen zu à 3 g gegossen.

Beispiel 28
Vaginaltabletten

Herstellung einer Charge von 100 000 Vaginaltabletten à 90 mg Wirkstoff:

90 kg Milchzucker und 17 kg Carboxymethylcellulose werden gemischt, mit einer Lösung von 9 kg 2,2'-Dihydroxy-5,5'-methylendi-(p-toluolsulfonsäure) in 50 l vergälltem Alkohol befeuchtet und durch ein Sieb der lichten Maschenweite von 1,5 mm granuliert. Das Granulat wird im Wirbelschichttrockner bis zu einer relativen Feuchte von 50 bis 60% getrocknet, mit 4 kg Talkum vermischt und nach dem Sieben zu Tabletten à 1200 mg verpresst.

Beispiel 29
Gel

1 g Gel enthält 18 mg Wirkstoff; Herstellung einer Charge von 25 kg:

18,750 kg Polyethylenglykol 400 und 5,000 kg Polyethylenglykol 20 000 werden bei 60° bis 70 °C geschmolzen. In die Schmelze wird die 36%ige Lösung von 2,2'-Dihydroxy-5,5'-methylendi-(p-toluolsulfonsäure) eingerührt. Unter gutem Rühren wird das Gel auf 20 °C abgekühlt und in Tuben à 50 g Inhalt abgefüllt.

Beispiel 30
Salbe

1 g Salbe enthält 50 mg Wirkstoff; Herstellung einer Charge von 25 kg:

8,750 kg Polyethylenglykol 400 und 13,750 kg Polyethylenglykol 550 werden bei 50 °C geschmolzen. Nach dem Abkühlen auf 40 °C werden 1,250 kg 2,2'-Dihydroxy-5,5'-methylendi-(p-toluolsulfonsäure), gelöst in der gleichen Menge Wasser, eingetragen. Man rührt, bis die Salbe eine Temperatur von 25 °C erreicht hat. Die Salbe wird in Tuben à 50 g Inhalt abgefüllt.

Beispiel 31
Suppositorien

1 Suppositorium enthält 100 mg Wirkstoff, Herstellung einer Charge von 10 000 Stück:

1 kg 2,2'-Dihydroxy-5,5'-methylendi-(p-toluolsulfonsäure)-diharnstoffsalz wird mit 0,1 kg kolloidalem Siliziumdioxid auf eine Teilchengrösse kleiner als 30 µm vermahlen und in 18,9 kg bei 40 °C geschmolzenes Hartfett (Adeps solidus) durch Rühren eingearbeitet und in Suppositorien vergossen.

**Patentansprüche**

für die Vertragsstaaten: DE, FR, IT, NL, BE, CH, GB, SE, LI, LU

1. 2,2'-Dihydroxy-5,5'-methylendi-(p-toluolsulfonsäure) und ihre Salze.

2. 2,2'-Dihydroxy-5,5'-methylendi-(p-toluolsulfonsäure) und deren Salze, die in Wasser von 23 °C zu mehr als 10 g/100 ml löslich sind.

3. 2,2'-Dihydroxy-5,5'-methylendi-(p-toluolsulfonsäure) und deren Natrium-, Ammonium- und Harnstoffsalz.

4. Verfahren zur Herstellung von 2,2'-Dihydroxy-5,5'-methylendi-(p-toluolsulfonsäure) und deren Salze, dadurch gekennzeichnet, dass man

a) m-Kresol-6-sulfonsäure und/oder deren Salze in saurem Medium mit Formaldehyd und/oder Formaldehyd abspaltenden Mitteln umsetzt und gewünschtenfalls ein erhaltenes Salz in die freie Säure oder ein anderes Salz überführt.

b) m-Kresol-6-sulfonsäure und/oder deren Salze in basischem Medium mit Formaldehyd und/ oder Formaldehyd abspaltenden Mitteln in 4-Hydroxymethyl-m-kresol-6-sulfonsäure überführt und diese in saurem Medium mit m-Kresol-6-sulfonsäure und/oder deren Salzen kondensiert und gewünschtenfalls ein erhaltenes Salz in die freie Säure oder ein anderes Salz überführt, oder

c) in 6-Stellung mit einem abspaltbaren Substituenten substituiertes m-Kresol in saurem Medium mit Formaldehyd und/oder Formaldehyd abspaltenden Mitteln zu 5,5'-disubstituiertem 4,4'-Dihydroxy-2,2'-dimethyldiphenylmethan umsetzt, die Substituenten in 5- und 5'-Stellung abspaltet und in saurem Medium mit Schwefelsäure und/ oder einem Sulfonsäuregruppen abspaltenden Mittel sulfoniert und gegebenenfalls ein erhaltenes Salz in die freie Säure oder ein anderes Salz überführt.

5. Verfahren nach Anspruch 4, dadurch gekennzeichnet, dass man

a) m-Kresol-6-sulfonsäure, deren Ammonium- oder Alkalisalz in Gegenwart von organischen Aminen, vorzugsweise Diaminen, oder die Salze von m-Kresol-6-sulfonsäure mit organischen Aminen, vorzugsweise Diaminen, in saurem Medium mit Formaldehyd und/oder Formaldehyd abspaltenden Mitteln umsetzt und gewünschtenfalls ein erhaltenes Salz in die freie Säure oder ein anderes Salz überführt,

b) m-Kresol-6-sulfonsäure, deren Ammonium- oder Natriumsalz in Gegenwart von organischen Aminen, vorzugsweise Diaminen, oder die Salze von m-Kresol-6-sulfonsäure mit organischen Aminen, vorzugsweise Diaminen, in basischem Medium mit Formaldehyd und/oder Formaldehyd abspaltenden Mitteln in ein Salz von 4-Hydroxymethyl-m-kresol-6-sulfonsäure mit organischen Aminen, vorzugsweise Diaminen, überführt und dieses in saurem Medium mit m-Kresol-6-sulfonsäure, deren Ammonium- oder Natriumsalz in Gegenwart von organischen Aminen, vorzugsweise Diaminen, oder mit einem Salz von m-Kresol-6-sulfonsäure mit einem organischen Amin, vorzugsweise Diamin, kondensiert und gewünschtenfalls ein erhaltenes Salz in die freie Säure oder ein anderes Salz überführt, oder

c) in 6-Stellung mit Chlor, Brom oder Jod substituiertes m-Kresol in saurem Medium mit Formaldehyd und/oder Formaldehyd abspaltenden Mitteln zu 5,5'-Dichlor-, -brom- oder -iod-4,4'-dihydroxy-2,2'-dimethyldiphenylmethan umsetzt, die Substituenten in 5- und 5'-Stellung durch katalytische Hydrierung abspaltet und in saurem Medium mit Schwefelsäure und/oder einem Sulfonsäuregruppen abspaltendem Mittel sulfoniert und gewünschtenfalls ein erhaltenes Salz in die freie Säure oder ein anderes Salz überführt.

6. Verfahren nach Anspruch 5, dadurch gekennzeichnet, dass man

a) m-Kresol-6-sulfonsäure, deren Ammonium- oder Natriumsalz in Gegenwart von Tetramethylethylendiamin oder das Salz der m-Kresol-6-sulfonsäure mit Tetramethylethylendiamin in saurem Medium mit Formaldehyd umsetzt und gewünschtenfalls das erhaltene Salz, gewünschtenfalls über das Natriumsalz, in die freie Säure oder ein anderes Salz überführt,

b) m-Kresol-6-sulfonsäure, deren Ammonium- oder Natriumsalz in Gegenwart von Tetramethylethylendiamin oder das Salz der m-Kresol-6-sulfonsäure mit Tetramethylethylendiamin in Gegenwart von Alkalihydroxid mit Formaldehyd in das Tetramethylethylendiaminsalz von 4-Hydroxymethyl-m-kresol-6-sulfonsäure überführt und dieses in saurem Medium mit m-Kresol-6-sulfonsäure, deren Ammonium- oder Natriumsalz in Gegenwart von Tetramethylethylendiamin oder mit dem Tetramethylethylendiaminsalz der m-Kresol-6-sulfonsäure kondensiert und gewünschtenfalls das erhaltene Salz, gewünschtenfalls über das Natriumsalz, in die freie Säure oder ein anderes Salz überführt, oder

c) 6-Chlor-m-kresol in saurem Medium mit Formaldehyd und/oder Formaldehyd abspaltenden Mitteln zu 5,5'-Dichlor-4,4'-dihydroxy-2,2'-dimethyldiphenylmethan umsetzt, dieses durch Hydrierung am Platinkatalysator in 4,4'-Dihydroxy-2,2'-dimethyldiphenylmethan überführt und in saurem Medium mit konzentrierter Schwefelsäure sulfoniert.

7. Arzneimittel enthaltend 2,2'-Dihydroxy-5,5'-methylendi-(p-toluolsulfonsäure) und/oder deren pharmakologisch verträgliche Salze.

8. 2,2'-Dihydroxy-5,5'-methylendi-(p-toluolsulfonsäure) und/oder deren pharmakologisch verträgliche Salze mit organischen und anorganischen Basen zur Anwendung bei der Behandlung von krankem Gewebe.

9. Salze der 2,2'-Dihydroxy-5,5'-methylendi-(p-toluolsulfonsäure), deren Löslichkeit in Wasser bei 23 °C weniger als 3 g/100 ml beträgt.

10. Salze nach Anspruch 9, dadurch gekennzeichnet, dass es sich um Salze mit organischen Aminen handelt.

11. Salze nach Anspruch 10, dadurch gekennzeichnet, dass es sich um organische Diamine handelt.

12. Salze nach Anspruch 11, dadurch gekennzeichnet, dass in den Diaminen die beiden Aminogruppen durch eine, zwei oder drei $C_2$ bis $C_4$-Alkylenketten verbunden sind.

13. Salze nach einem der Ansprüche 11 oder 12, dadurch gekennzeichnet, dass das organische Diamin Piperazin, 1,4-Diazabicyclo[2.2.2]oktan, 1,4-Dimethylpiperazin, 1-Benzyl-4-methylpiperazin, Ethylendiamin oder Tetramethylethylendiamin ist.

14. Verfahren zur Herstellung von Salzen der 2,2'-Dihydroxy-5,5'-methylendi-(p-toluolsulfonsäure) mit organischen Aminen, dadurch gekennzeichnet, dass

a) 2,2'-Dihydroxy-5,5'-methylendi(p-toluolsulfonsäure) oder eines ihrer Salze mit dem organischen Amin umgesetzt, oder

b) m-Kresol-6-sulfonsäure oder deren Ammonium- oder Alkalisalz in Gegenwart von organischen Aminen oder ein Salz von m-Kresol-6-sulfonsäure mit organischen Aminen in saurem Medium mit Formaldehyd und/oder Formaldehyd abspaltenden Mitteln umgesetzt wird.

**Patentansprüche**

für den Vertragsstaat: AT

1. Verfahren zur Herstellung von 2,2'-Dihydroxy-5,5'-methylendi-(p-toluolsulfonsäure) und deren Salze, dadurch gekennzeichnet, dass man

a) m-Kresol-6-sulfonsäure und/oder deren Salze in saurem Medium mit Formaldehyd und/oder Formaldehyd abspaltenden Mitteln umsetzt und gewünschtenfalls ein erhaltenes Salz in die freie Säure oder ein anderes Salz überführt,

b) m-Kresol-6-sulfonsäure und/oder deren Salze in basischem Medium mit Formaldehyd und/oder Formaldehyd abspaltenden Mitteln in 4-Hydroxymethyl-m-kresol-6-sulfonsäure überführt und diese in saurem Medium mit m-Kresol-6-sulfonsäure und/oder deren Salzen kondensiert und gewünschtenfalls ein erhaltenes Salz in die freie Säure oder ein anderes Salz überführt, oder

c) in 6-Stellung mit einem abspaltbaren Substituenten substituiertes m-Kresol in saurem Medium mit Formaldehyd und/oder Formaldehyd abspaltenden Mitteln zu 5,5'-disubstituiertem 4,4'-Dihydroxy-2,2'-dimethyldiphenylmethan umsetzt, die Substituenten in 5- und 5'-Stellung abspaltet und in saurem Medium mit Schwefelsäure und/oder einem Sulfonsäuregruppen abspaltenden Mittel sulfoniert und gegebenenfalls ein erhaltenes Salz in die freie Säure oder ein anderes Salz überführt.

2. Verfahren nach Anspruch 2, dadurch gekennzeichnet, dass man

a) m-Kresol-6-sulfonsäure, deren Ammonium- oder Alkalisalz in Gegenwart von organischen Aminen, vorzugsweise Diaminen, oder die Salze von m-Kresol-6-sulfonsäure mit organischen Aminen, vorzugsweise Diaminen, in saurem Medium mit Formaldehyd und/oder Formaldehyd abspaltenden Mitteln umsetzt und gewünschtenfalls ein erhaltenes Salz in die freie Säure oder ein anderes Salz überführt,

b) m-Kresol-6-sulfonsäure, deren Ammonium- oder Natriumsalz in Gegenwart von organischen Aminen, vorzugsweise Diaminen, oder die Salze von m-Kresol-6-sulfonsäure mit organischen Aminen, vorzugsweise Diaminen, in basischem Medium mit Formaldehyd und/oder Formaldehyd abspaltenden Mitteln in ein Salz von 4-Hydroxymethyl-m-kresol-6-sulfonsäure mit organischen Aminen, vorzugsweise Diaminen, überführt und dieses in saurem Medium mit m-Kresol-6-sulfonsäure, deren Ammonium- oder Natriumsalz in Gegenwart von organischen Aminen, vorzugsweise Diaminen, oder mit einem Salz von m-Kresol-6-sulfonsäure mit einem organischen Amin, vorzugsweise Diamin, kondensiert und gewünschtenfalls ein erhaltenes Salz in die freie Säure oder ein anderes Salz überführt, oder

c) in 6-Stellung mit Chlor, Brom oder Jod substituiertes m-Kresol in saurem Medium mit Formaldehyd und/oder Formaldehyd abspaltenden Mitteln zu 5,5'-Dichlor-, -brom- oder -iod-4,4'-dihydroxy-2,2'-dimethyldiphenylmethan umsetzt, die Substituenten in 5- und 5'-Stellung durch katalytische Hydrierung abspaltet und in saurem Medium mit Schwefelsäure und/oder einem Sulfonsäuregruppen abspaltenden Mittel sulfoniert und gewünschtenfalls ein erhaltenes Salz in die freie Säure oder ein anderes Salz überführt.

3. Verfahren nach Anspruch 2, dadurch gekennzeichnet, dass man

a) m-Kresol-6-sulfonsäure, deren Ammonium- oder Natriumsalz in Gegenwart von Tetramethylethylendiamin oder das Salz der m-Kresol-6-sulfonsäure mit Tetramethylethylendiamin in saurem Medium mit Formaldehyd umsetzt und gewünschtenfalls das erhaltene Salz, gewünschtenfalls über das Natriumsalz, in die freie Säure oder ein anderes Salz überführt,

b) m-Kresol-6-sulfonsäure, deren Ammonium- oder Natriumsalz in Gegenwart von Tetramethylethylendiamin oder das Salz der m-Kresol-6-sulfonsäure mit Tetramethylethylendiamin in Gegenwart von Alkalihydroxid mit Formaldehyd in das Tetramethylethylendiaminsalz von 4-Hydroxymethyl-m-kresol-6-sulfonsäure überführt und dieses in saurem Medium mit m-Kresol-6-sulfonsäure, deren Ammonium- oder Natriumsalz in Gegenwart von Tetramethylethylendiamin oder mit dem Tetramethylethylendiaminsalz der m-Kresol-6-sulfonsäure kondensiert und gewünschtenfalls das erhaltene Salze, gewünschtenfalls über das Natriumsalz, in die freie Säure oder ein anderes Salz überführt, oder

c) 6-Chlor-m-kresol in saurem Medium mit Formaldehyd und/oder Formaldehyd abspaltenden Mitteln zu 5,5'-Dichlor-4,4'-dihydroxy-2,2'-dimethyldiphenylmethan umsetzt, dieses durch Hydrierung am Platinkatalysator in 4,4'-Dihydroxy-2,2'-dimethyldiphenylmethan überführt und in saurem Medium mit konzentrierter Schwefelsäure sulfoniert.

4. Verfahren nach einem der Ansprüche 1 bis 3, dadurch gekennzeichnet, dass man die Überführung erhaltener Salze in die freie Säure oder in andere Salze mittels eines Kationenaustauschers bewerkstelligt.

5. Verfahren zur Herstellung von Salzen der 2,2'-Dihydroxy-5,5'-methylendi-(p-toluolsulfonsäure) mit organischen Aminen, dadurch gekennzeichnet, dass

a) 2,2'-Dihydroxy-5,5'-methylendi-(p-toluolsulfonsäure) oder eines ihrer Salze mit dem organischen Amin umgesetzt, oder

b) m-Kresol-6-sulfonsäure oder deren Ammonium- oder Alkalisalz in Gegenwart von organischen Aminen oder ein Salz von m-Kresol-6-sulfonsäure mit organischen Aminen in saurem Medium mit Formaldehyd und/oder Formaldehyd abspaltenden Mitteln umgesetzt wird.

6. Verfahren zur Herstellung von Salzen der 2,2'-Dihydroxy-5,5'-methylendi-(p-toluolsulfonsäure) mit organischen Diaminen, dadurch gekennzeichnet, dass

a) 2,2'-Dihydroxy-5,5'-methylendi-(p-toluolsulfonsäure) oder eines ihrer Salze mit einem organischen Diamin umgesetzt, oder

b) m-Kresol-6-sulfonsäure oder deren Ammonium- oder Alkalisalz in Gegenwart von organischen Diaminen oder ein Salz von m-Kresol-6-sulfonsäure mit organischen Diaminen in saurem Medium mit Formaldehyd und/oder Formaldehyd abspaltenden Mitteln umgesetzt wird.

7. Verfahren nach Anspruch 6, dadurch gekennzeichnet, dass es sich bei den organischen Diaminen um Piperazin, 1,4-Diazabicyclo[-2.2.2]-oktan, 1,4-Dimethylpiperazin, 1-Benzyl-4-methylpiperazin, Ethylendiamin oder Tetramethylethylendiamin handelt.

**Revendications**

pour les Etats contractants: DE, FR, IT, NL, BE, CH, GB, SE, LI, LU

1. Acide 2,2'-dihydroxy-5,5'-méthylène-bis-(p-toluènesulfonique) et ses sels.

2. Acide 2,2'-dihydroxy-5,5'-méthylène-bis-(p-toluènesulfonique) et ses sels qui sont solubles dans l'eau à 23 °C à raison de plus de 10 g/100 ml.

3. Acide 2,2'-dihydroxy-5,5'-méthylène-bis-(p-toluènesulfonique) et ses sels de sodium, d'ammonium et d'urée.

4. Procédé de préparation du composé constitué par l'acide 2,2'-dihydroxy-5,5'-méthylène-bis-(p-toluènesulfonique) et de ses sels, qui est caractérisé par le fait que:

a) on fait réagir en milieu acide l'acide m-crésol-6-sulfonique et/ou ses sels avec le formaldéhyde et/ou des agents libérant du formaldéhyde et si on le désire, on convertit un sel obtenu en l'acide libre ou en un autre sel,

b) on convertit en milieu basique l'acide m-crésol-6-sulfonique et/ou ses sels, avec le formaldéhyde et/ou des agents libérant du formaldéhyde en acide 4-hydroxyméthyl-m-crésol-6-sulfonique et on condense celui-ci en milieu acide avec l'acide m-crésol-6-sulfonique et/ou ses sels et si on le désire, on convertit un sel obtenu en l'acide libre ou en un autre sel, ou

c) on fait réagir en milieu acide un m-crésol substitué en position 6 un substituant éliminable, avec le formaldéhyde et/ou des agents libérant du formaldéhyde, pour obtenir un 4,4'-dihydroxy-2,2'-diméthyl-diphénylméthane disubstitué en 5,5'-, on élimine les substituants en positions 5 et 5' et on sulfone en milieu acide au moyen d'acide sulfurique et/ou d'un agent libérant des groupes acide sulfonique et si on le désire, on convertit un sel obtenu en l'acide libre ou en un autre sel.

5. Procédé selon la revendication 4, caractérisé par le fait que:

a) on fait réagir en milieu acide l'acide m-crésol-6-sulfonique, son sel d'ammonium ou son sel alcalin, en présence d'amines organiques, de préférence de diamines, ou les sels de l'acide m-crésol-6-sulfonique et d'amines organiques, de préférence de diamines, avec le formaldéhyde et/ou avec des agents libérant du formaldéhyde et si on le désire, on convertit un sel obtenu en l'acide libre ou en autre sel,

b) on convertit en milieu basique l'acide m-crésol-6-sulfonqiue, son sel d'ammonium ou son sel de sodium, en présence d'amines organiques, de préférence de diamines, ou les sels de l'acide m-crésol-6-sulfonique et d'amines organiques, de préférence de diamines, avec le formaldéhyde et/ou des agents libérant du formaldéhyde, en un sel de l'acide 4-hydroxyméthyl-m-crésol-6-sulfonique et d'amines organiques, de préférence de diamines, et on condense celui-ci en milieu acide avec l'acide m-crésol-6-sulfonique, son sel d'ammonium ou son sel de sodium, en présence d'amines organiques, de préférence de diamines, ou avec un sel de l'acide m-crésol-6-sulfonique et d'une amine organique, de préférence d'une diamine, et si on le désire, on convertit un sel obtenu en l'acide libre ou en un autre sel, ou

c) on fait réagir en milieu acide un m-crésol substitué en position 6 par du chlore, du brome, ou de l'iode, avec le formaldéhyde et/ou des agens libérant de formaldéhyde pour obtenir le 5,5'-dichloro-, le 5,5'-bromo- ou le 5,5'-iodo-4,4'-dihydroxy-2,2'-diméthyl-diphénylméthane, on élimine par hydrogénation catalytique les substituants en positions 5 et 5' et on sulfone en milieu acide, à l'aide d'acide sulfurique et/ou d'un agent libérant des groupes acide sulfonique, et si on le désire, on convertit un sel obtenu en l'acide libre ou en un autre sel.

6. Procédé selon la revendication 5, caractérisé en ce que:

a) on fait réagir en milieu acide l'acide m-crésol-6-sulfonique, son sel d'ammonium ou son sel de sodium, en présence de tétraméthyléthylènediamine, ou le sel de l'acide m-crésol-6-sulfonique et de la tétraméthyléthylènediamine, avec le formaldéhyde et si on le désire, on convertit le sel obtenu, en passant si on le désire, par le sel de sodium, en l'acide libre ou en un autre sel,

b) on convertit l'acide m-crésol-6-sulfonique, son sel d'ammonium ou son sel de sodium, en présence de tétraméthyléthylènediamine, ou le sel de l'acide m-crésol-6-sulfonique et de la tétraméthyléthylène diamine en présence d'hydroxyde alcalin, avec le formaldéhyde, en sel de tétraméthyléthylènediamine de l'acide 4-hydroxyméthyl-m-crésol-6-sulfonique et on condense ce sel en milieu acide avec l'acide m-crésol-6-sulfonique, son sel d'ammonium ou son sel de sodium, en présence de tétraméthyléthylènediamine, ou avec le sel de tétraméthyléthylènediamine de l'acide m-crésol-6-sulfonique et si on le désire on convertit le sel obtenu, en passant si on le désire, par le sel de sodium, en l'acide libre ou en un autre sel, ou

c) on fait réagir le 6-chloro-m-crésol, en milieu acide, avec le formaldéhyde et/ou des agents libérant du formaldéhyde, pour obtenir le 5,5'-dichloro-4,4'-dihydroxy-2,2'-diméthyldiphénylméthane, on convertit celui-ci, par hydrogénation sur un catalyseur au platine, en 4,4'-dihydroxy-2,2'-diméthyldiphénylméthane et on sulfone en milieu acide à l'aide d'acide sulfurique concentré.

7. Médicament contenant de l'acide 2,2'-dihydroxy-5,5'-méthylène-bis-(p-toluènesulfonique) et/ou ses sels pharmacologiquement compatibles.

8. Acide 2,2'-dihydroxy-5,5'-méthylène-bis-(p-toluènesulfonique) et/ou ses sels pharmacologiquement compatibles de bases organiques et minérales, pour l'application dans le traitement de tissu malade.

9. Sels de l'acide 2,2'-dihydroxy-5,5'-méthylène-bis-(p-toluènesulfonique) dont la solubilité dans l'eau à 23 °C est inférieure à 3 g/100 ml.

10. Sels selon la revendication 9, caractérisés en ce qu'il s'agit de sels d'amines organiques.

11. Sels selon la revendication 10, caractérisés en ce qu'il s'agit de diamines organiques.

12. Sels selon la revendication 11, caractérisés en ce que dans les diamines, les deux groupes amine sont reliés par une, deux ou trois chaînes alkylène en $C_2$ à $C_4$.

13. Sels selon l'une des revendications 11 ou 12, caractérisés par le fait que la diamine organique est la pipérazine, le 1,4-diazabicyclo[2,2,2]octane, la 1,4-diméthylpipérazine, la 1-benzyl-4-méthylpipérazine, l'éthylènediamine ou la tétraméthylènediamine.

14. Procédé de préparation des sels de l'acide 2,2'-dihydroxy-5,5'-méthylène-bis-(p-toluènesulfonique) et d'amines organiques, caractérisé en ce que:

a) on fait réagir l'acide 2,2'-dihydroxy-5,5'-méthylène-bis-(p-toluènesulfonique) ou l'un de ses sels avec l'amine organique, ou

b) on fait réagir l'acide m-crésol-6-sulfonique ou son sel d'ammonium ou de métal alcalin, en présence d'amines organiques, ou un sel de l'acide m-crésol-6-sulfonique et d'amines organiques, en milieu acide, avec le formaldéhyde et/ou avec des agents libérant du formaldéhyde.

**Revendications**

pour l'Etat contractant AT

1. Procédé de préparation du composé constitué par l'acide 2,2'-dihydroxy-5,5'-méthylène-bis-(p-toluènesulfonique) et de ses sels, qui est caractérisé par le fait que:

a) on fait réagir en milieu acide l'acide m-crésol-6-sulfonique et/ou ses sels avec le formaldéhyde et/ou des agents libérant du formaldéhyde et si on le désire, on convertit un sel obtenu en l'acide libre ou en un autre sel,

b) on convertit en milieu basique l'acide m-crésol-6-sulfonique et/ou ses sels, avec le formaldéhyde et/ou des agents libérant du formaldéhyde en acide 4-hydroxyméthyl-m-crésol-6-sulfonique et on condense celui-ci en milieu acide avec l'acide m-crésol-6-sulfonique et/ou ses sels et si on le désire, on convertit un sel obtenu en l'acide libre ou en un autre sel, ou

c) on fait réagir en milieu acide un m-crésol substitué en position 6 un substituant éliminable,

avec le formaldéhyde et/ou des agents libérant du formaldéhyde, pour obtenir un 4,4'-dihydroxy-2,2'-diméthyl-diphénylméthane disubstitué en 5,5'-, on élimine les substituants en positions 5 et 5' et on sulfone en milieu acide au moyen d'acide sulfurique et/ou d'un agent libérant des groupes acide sulfonique et si on le désire, on convertit un sel obtenu en l'acide libre ou en un autre sel.

2. Procédé selon la revendication 1, caractérisé par le fait que:

a) on fait réagir en milieu acide l'acide m-crésol-6-sulfonique, son sel d'ammonium ou son sel alcalin, en présence d'amines organiques, de préférence de diamines, ou les sels de l'acide m-crésol-6-sulfonique et d'amines organiques, de préférence de diamines, avec le formaldéhyde et/ou avec des agents libérant du formaldéhyde et si on le désire, on convertit un sel obtenu en l'acide libre ou en autre sel,

b) on convertit en milieu basique l'acide m-crésol-6-sulfonique, son sel d'ammonium ou son sel de sodium, en présence d'amines organiques, de préférence de diamines, ou les sels de l'acide m-crésol-6-sulfonique et d'amines organiques, de préférence de diamines, avec le formaldéhyde et/ou des agents libérant du formaldéhyde, en un sel de l'acide 4-hydroxyméthyl-m-crésol-6-sulfonique et d'amines organiques, de préférence de diamines, et on condense celui-ci en milieu acide avec l'acide m-crésol-6-sulfonique, son sel d'ammonium ou son sel de sodium, en présence d'amines organiques, de préférence de diamines, ou avec un sel de l'acide m-crésol-6-sulfonique et d'une amine organique, de préférence d'une diamine, et si on le désire, on convertit un sel obtenu en l'acide libre ou en un autre sel, ou

c) on fait réagir en milieu acide un m-crésol substitué en position 6 par du chlore, du brome, ou de l'iode, avec le formaldéhyde et/ou des agents libérant du formaldéhyde pour obtenir le 5,5'-dichloro-, 5,5'-bromo- ou le 5,5'-iodo-4,4'-dihydroxy-2,2'-diméthyl-diphénylméthane, on élimine par hydrogénation catalytique les substituants en positions 5 et 5' et on sulfone en milieu acid, à l'aide d'acide sulfurique et/ou d'un agent libérant des groupes acide sulfonique, et si on le désire, on convertit un sel obtenu en l'acide libre ou en un autre sel.

3. Procédé selon la revendication 2, caractérisé en ce que:

a) on fait réagir en milieu acide l'acide m-crésol-6-sulfonique, son sel d'ammonium ou son sel de sodium, en présence de tétraméthyléthylènediamine, ou le sel de l'acide m-crésol-6-sulfonique et de la tétraméthyléthylènediamine, avec le formaldéhyde et si on le désire, on convertit le sel obtenu, en passant si on le désire, par le sel de sodium, en l'acide libre ou en un autre sel,

b) on convertit l'acide m-crésol-6-sulfonique, son sel d'ammonium ou son sel de sodium, en présence de tétraméthyléthylènediamine, ou le sel de l'acide m-crésol-6-sulfonique et de la tétraméthyléthylène diamine en présence d'hydroxyde alcalin, avec le formaldéhyde, en sel de tétraméthyléthylènediamine de l'acide 4-hydroxyméthyl-m-crésol-6-sulfonique et on condense ce sel en milieu acide avec l'acide m-crésol-6-sulfonique, son sel d'ammonium ou son sel de sodium, en présence de tétraméthyléthylènediamine, ou avec le sel de tétraméthyléthylènediamine de l'acide m-crésol-6-sulfonique et si on le désire on convertit le sel obtenu, en passant si on le désire, par le sel de sodium, en l'acide libre ou en un autre sel, ou

c) on fait réagir le 6-chloro-m-crésol, en milieu acide, avec le formaldéhyde et/ou des agents libérant du formaldéhyde, pour obtenir le 5,5'-dichloro-4,4'-dihydroxy-2,2'-diméthyldiphénylméthane, on convertit celui-ci, par hydrogénation sur un catalyseur au platine, en 4,4'-dihydroxy-2,2'-diméthyldiphénylméthane et on sulfone en milieu acide à l'aide d'acide sulfurique concentré.

4. Procédé selon l'une des Revendication 1, 2 ou 3, caractérisé par le fait qu'on convertit des sels obtenus en l'acide libre ou en autres sels à l'aide d'échangeurs d'ions acides.

5. Procédé de préparation des sels de l'acide 2,2'-dihydroxy-5,5'-méthylène-bis-(p-toluènesulfonique) et d'amines organiques, caractérsié en ce que:

a) on fait réagir l'acide 2,2'-dihydroxy-5,5'-méthylène-bis-(p-toluènesulfonique) ou l'un de ses sels avec l'amine organique, ou

b) on fait réagir l'acide m-crésol-6-sulfonique ou son sel d'ammonium ou de métal alcalin, en présence d'amines organiques, ou un sel de l'acide m-crésol-6-sulfonique et d'amines organiques, en milieu acide, avec le formaldéhyde et/ou avec des agents libérant du formaldéhyde.

6. Procédé de préparation des dels de l'acide 2,2'-dihydroxy-5,5'-méthylène-bis-(p-toluènesulfonique) et d'amines organiques, caractérisé en ce que:

a) on fait réagir l'acide 2,2'-dihydroxy-5,5'-méthylène-bis-(p-toluènesulfonique) ou l'un de ses sels avec de diamines organiques, ou

b) on fait réagir l'acide 2,2'-dihydroxy-5,5'-méthylène-bis-(p-toluènesulfonique) ou son sel d'ammonium ou de métal alcalin, en présence de diamines organiques, ou un sel de l'acide m-crésol-6-sulfonique et de diamines organiques, en milieux acide avec le formaldéhyde et/ou avec des agents libérant du formaldéhyde.

7. Procédé selon la revendication 6, caractérisé par le fait que le diamine organique est la pipérazine, le 1,4-diazabicyclo[2.2.2]octane, la 1,4-diméthylpipérazine, la 1-benzyl-4-méthylpipérazine, l'éthylènediamine ou la tétraméthyléthylènediamine.

**Claims**

(for the Contracting states: DE, FR, IT, NL, BE, CH, GB, SE, LI, LU)

1. 2,2'-Dihydroxy-5,5'-methylenedi-(p-toluenesulphonic acid) and its salts.

2. 2,2'-Dihydroxy-5,5'-methylenedi-(p-toluenesulphonic acid) and salts thereof which are soluble in water at 23 °C to the extent of more than 10 g/100 ml.

3. 2,2'-Dihydroxy-5,5'-methylenedi-(p-toluenesulphonic acid) and its sodium, ammonium and urea salts.

4. Process for the preparation of 2,2'-dihydroxy-5,5'-methylenedi-(p-toluenesulphonic acid) and its salts, characterised in that

a) m-cresol-6-sulphonic acid and/or its salts are reacted in an acid medium with formaldehyde and/or formaldehyde donors and, if desired, a resulting salt is converted into the free acid or into another salt,

b) m-cresol-6-sulphonic acid and/or its salts are converted, in a basic medium, by means of formaldehyde and/or formaldehyde donors into 4-hydroxymethyl-m-cresol-6-sulphonic acid and the latter is subjected in an acid medium to a condensation reaction with m-cresol-6-sulphonic acid and/or its salts and, if desired, a resulting salt is converted into the free acid or into another salt, or

c) m-cresol which is substituted in the 6-position by a detachable substituent is reacted, in an acid medium, with formaldehyde and/or formaldehyde donors to give a 5,5'-disubstituted 4,4'-dihydroxy-2,2'-dimethyldiphenylmethane, the substituents in the 5-position and 5'-position are split off and the product is sulphonated in an acid medium with sulphuric acid and/or an agent which splits off sulphonic acid groups and, if appropriate, a resulting salt is converted into the free acid or into another salt.

5. Process according to Claim 4, characterised in that

a) m-cresol-6-sulphonic acid or its ammonium or alkali metal salt, in the presence of organic amines, preferably diamines, or the salts of m-cresol-6-sulphonic acid with organic amines, preferably diamines, are reacted, in an acid medium, with formaldehyde and/or formaldehyde donors and, if desired, a resulting salt is converted into the free acid or into another salt,

b) m-cresol-6-sulphonic acid or its ammonium or sodium salt, in the presence of organic amines, preferably diamines, or the salts of m-cresol-6-sulphonic acid with organic amines, preferably diamines, are converted, in a basic medium, by means of formaldehyde and/or formaldehyde donors, into a salt of 4-hydroxymethyl-m-cresol-6-sulphonic acid with organic amines, preferably diamines, and this salt is subjected, in an acid medium, to a condensation reaction with m-cresol-6-sulphonic acid or its ammonium or sodium salt in the presence of organic amines, preferably diamines or to a condensation reaction with a salt of m-cresol-6-sulphonic acid with an organic amine, preferably a diamine, and, if desired, a resulting salt is converted into the free acid or into another salt, or

c) m-cresol which is substituted in the 6-position by chlorine, bromine or iodine is reacted, in an acid medium, with formaldehyde and/or formaldehyde donors to give 5,5'-dichloro-4,4'-dihydroxy-2,2'-dimethyldiphenylmethane, 5,5'-dibromo-4,4'-dihydroxy-2,2'-dimethyldiphenylmethane or 5,5'-diiodo-4,4'-dihydroxy-2,2'-dimethyldiphenylmethane, the substituents in the 5-position and 5'-position are split off by catalytic hydrogenation and the product is sulphonated, in an acid medium, with sulphuric acid and/or an agent which splits off sulphonic acid groups, and, if desired, a resulting salt is converted into the free acid or into another salt.

6. Process according to Claim 5, characterised in that

a) m-cresol-6-sulphonic acid or its ammonium or sodium salt in the presence of tetramethylethylenediamine, or the salt of m-cresol-6-sulphonic acid with tetramethylethylenediamine, is reacted with formaldehyde in an acid medium, and, if desired, the resulting salt is converted, if desired via the sodium salt, into the free acid or into another salt,

b) m-cresol-6-sulphonic acid or its ammonium or sodium salt in the presence of tetramethylethylenediamine, or the salt of m-cresol-6-sulphonic acid with tetramethylethylenediamine in the presence of an alkali metal hydroxide, is converted by means of formaldehyde into the tetramethylethylenediamine salt of 4-hydroxymethyl-m-cresol-6-sulphonic acid and this salt is subjected, in an acid medium, to a condensation reaction with m-cresol-6-sulphonic acid or its ammonium or sodium salt in the presence of tetramethylethylenediamine, or to a condensation reaction with the tetramethylethylenediamine salt of m-cresol-6-sulphonic acid, and, if desired, the resulting salt is converted, if desired via the sodium salt, into the free acid or into another salt, or

c) 6-chloro-m-cresol is reacted, in an acid medium, with formaldehyde and/or formaldehyde donors to give 5,5'-dichloro-4,4'-dihydroxy-2,2'-dimethyldiphenylmethane, the latter is converted by hydrogenation over a platinum catalyst into 4,4'-dihydroxy-2,2'-dimethyldiphenylmethane and the latter is sulphonated in an acid medium with concentrated sulphuric acid.

7. Medicaments containing 2,2'-dihydroxy-5,5'-methylenedi-(p-toluenesulphonic acid) and/or its pharmacologically acceptable salts.

8. 2,2'-Dihydroxy-5,5'-methylenedi(p-toluenesulphonic acid) and/or its pharmacologically acceptable salts with organic and inorganic bases for use in a method for treatment of diseased tissue.

9. Salts of 2,2'-dihydroxy-5,5'-methylenedi-(p-toluenesulphonic acid) which have a solubility in water at 23 °C of less than 3 g/100 ml.

10. Salts according to Claim 9, characterised in that they are salts with organic amines.

11. Salts according to Claim 10, characterised in that they are salts with organic diamines.

12. Salts according to Claim 11, characterised in that the two amino groups in the diamines are linked by one, two or three $C_2$ to $C_4$ alkylene chains.

13. Salts according to one of Claims 11 or 12, characterised in that the organic diamine is piperazine, 1,4-diazabicyclo[2.2.2]octane, 1,4-dimethylpiperazine, 1-benzyl-4-methylpiperazine, ethylenediamine or tetramethylethylenediamine.

14. Process for the preparation of salts of 2,2'-dihydroxy-5,5'-methylenedi-(p-toluenesulphonic

acid) with organic amines, characterised in that

a) 2,2'-dihydroxy-5,5'-methylenedi-(p-toluenesulphonic acid) or one of its salts is reacted with the organic amine, or

b) m-cresol-6-sulphonic acid or its ammonium or alkali metal salt in the presence of organic amines, or a salt of m-cresol-6-sulphonic acid with organic amines, is reacted, in an acid medium, with formaldehyde and/or formaldehyde donors.

**Claims**

(for the Contracting state: AT)

1. Process for the preparation of 2,2'-dihydroxy-5,5'-methylenedi-(p-toluenesulphonic acid) and its salts, characterised in that

a) m-cresol-6-sulphonic acid and/or its salts are reacted in an acid medium with formaldehyde and/or formaldehyde donors and, if desired, a resulting salt is converted into the free acid or into another salt,

b) m-cresol-6-sulphonic acid and/or its salts are converted, in a basic medium, by means of formaldehyde and/or formaldehyde donors into 4-hydroxymethyl-m-cresol-6-sulphonic acid and the latter is subjected in an acid medium to a condensation reaction with m-cresol-6-sulphonic acid and/or its salts and, if desired, a resulting salt is converted into the free acid or into another salt, or

c) m-cresol which is substituted in the 6-position by a detachable substituent is reacted, in an acid medium, with formaldehyde and/or formaldehyde donors to give a 5,5'-disubstituted 4,4'-dihydroxy-2,2'-dimethyldiphenylmethane, the substituents in the 5-position and 5'-position are split off and the product is sulphonated in an acid medium with sulphuric acid and/or an agent which splits off sulphonic acid groups and, if appropriate, a resulting salt is converted into the free acid or into another salt.

2. Process according to Claim 1, characterised in that

a) m-cresol-6-sulphonic acid or its ammonium or alkali metal salt, in the presence of organic amines, preferably diamines, or the salts of m-cresol-6-sulphonic acid with organic amines, preferably diamines, are reacted, in an acid medium, with formaldehyde and/or formaldehyde donors and, if desired, a resulting salt is converted into the free acid or into another salt,

b) m-cresol-6-sulphonic acid or its ammonium or sodium salt, in the presence of organic amines, preferably diamines, or the salts of m-cresol-6-sulphonic acid with organic amines, preferably diamines, are converted, in a basic medium, by means of formaldehyde and/or formaldehyde donors, into a salt of 4-hydroxymethyl-m-cresol-6-sulphonic acid with organic amines, preferably diamines, and this salt is subjected, in an acid medium, to a condensation reaction with m-cresol-6-sulphonic acid or its ammonium or sodium salt in the presence of organic amines, preferably diamines or to a condensation reaction with a salt of m-cresol-6-sulphonic acid with an organic amine, preferably a diamine, and, if desired, a resulting salt is converted into the free acid or into another salt, or

c) m-cresol which is substituted in the 6-position by chlorine, bromine or iodine is reacted, in an acid medium, with formaldehyde and/or formaldehyde donors to give 5,5'-dichloro-4,4'-dihydroxy-2,2'-dimethyldiphenylmethane, 5,5'-dibromo-4,4'-dihydroxy-2,2'-dimethyldiphenylmethane or 5,5'-diiodo-4,4'-dihydroxy-2,2'-dimethyldiphenylmethane, the substituents in the 5-position and 5'-position are split off by catalytic hydrogenation and the product is sulphonated, in an acid medium, with sulphuric acid and/or an agent which splits off sulphonic acid groups, and, if desired, a resulting salt is converted into the free acid or into another salt.

3. Process according to Claim 2, characterised in that

a) m-cresol-6-sulphonic acid or its ammonium or sodium salt in the presence of tetramethylethylenediamine, or the salt of m-cresol-6-sulphonic acid with tetramethylethylenediamine, is reacted with formaldehyde in an acid medium, and, if desired, the resulting salt is converted, if desired via the sodium salt, into the free acid or into another salt,

b) m-cresol-6-sulphonic acid or its ammonium or sodium salt in the presence of tetramethylethylenediamine, or the salt of m-cresol-6-sulphonic acid with tetramethylethylenediamine in the presence of an alkali metal hydroxide, is converted by means of formaldehyde into the tetramethylethylenediamine salt of 4-hydroxymethyl-m-cresol-6-sulphonic acid and this salt is subjected, in an acid medium, to a condensation reaction with m-cresol-6-sulphonic acid or its ammonium or sodium salt in the presence of tetramethylethylenediamine, or to a condensation reaction with the tetramethylethylenediamine salt of m-cresol-6-sulphonic acid, and, if desired, the resulting salt is converted, if desired via the sodium salt, into the free acid or into another salt, or

c) 6-chloro-m-cresol is reacted, in an acid medium, with formaldehyde and/or formaldehyde donors to give 5,5'-dichloro-4,4'-dihydroxy-2,2'-dimethyldiphenylmethane, the latter is converted by hydrogenation over a platinum catalyst into 4,4'-dihydroxy-2,2'-dimethyldiphenylmethane and the latter is sulphonated in an acid medium with concentrated sulphuric acid.

4. Process according to one of Claims 1, 2 or 3, characterised in that obtained salts are converted into the free acid or into other salts with an acid ion exchanger.

5. Process for the preparation of salts of 2,2'-dihydroxy-5,5'-methylenedi-(p-toluenesulphonic acid) with organic amines, characterised in that

a) 2,2'-dihydroxy-5,5'-methylenedi-(p-toluenesulphonic acid) or one of its salts is reacted with the organic amine, or

b) m-cresol-6-sulphonic acid or its ammonium or alkali metal salt in the presence of organic amines, or a salt of m-cresol-6-sulphonic acid with organic amines, is reacted, in an acid medium with formaldehyde and/or formaldehyde donors.

6. Process for the preparation of salts of 2,2'-dihydroxy-5,5'-methylenedi-(p-toluenesulphonic acid) with organic diamines, characterised in that

a) 2,2'-dihydroxy-5,5'-methylenedi-(p-toluenesulphonic acid) or one of its salts is reacted with the organic diamine, or

b) m-cresol-6-sulphonic acid or its ammonium or alkali metal salt in the presence of organic diamines, or a salt of m-cresol-6-sulphonic acid with organic diamines, is reacted, in an acid medium, with formaldehyde and/or formaldehyde donors.

7. Process according to Claim 6, characterised in that the organic diamines are piperazine, 1,4-diazabicyclo[2.2.2]-octane, 1,4-dimethylpiperazine, 1-benzyl-4-methylpiperazine, ethylenediamine or tetramethylethylenediamine.